# EUROPEAN PATENT SPECIFICATION

(11) **EP 0 431 838 B1**
(45) Date of publication and mention of the grant of the patent: **25.10.1995**
(21) Application number: 90312983.1
(22) Date of filing: 29.11.1990
(51) Int. Cl.: C07D 211/76, C07D 223/10, C07D 205/08, C07D 263/24, C07D 207/273, C07D 265/32, C07D 207/27, C07D 263/22, C07D 265/10, A61K 49/04

(54) **Nonionic radiographic contrast agents**
Nichtionische radiographische Kontrastmittel
Agents de contraste radiographique non-ioniques

(30) Priority: 29.11.1989 US 442869
(43) Date of publication of application: 12.06.1991
(73) Proprietor: BRACCO INTERNATIONAL B.V., 1083 HJ Amsterdam (NL)
(72) Inventor: Ranganathan, Ramachandran S., Princeton, New Jersey (US); Marinelli, Edmund R., Lawrenceville, New Jersey (US); Arunachalam, Thangavel, Plainsboro, New Jersey (US); Pillai, Radhakrishna, Kendall Park, New Jersey (US)
(74) Representative: Chopard, Pierre-Antoine

(56) References cited:
- EP-A- 0 022 744
- EP-A- 0 026 281
- EP-A- 0 082 803
- EP-A- 0 105 752
- DE-A- 2 726 196
- DE-A- 3 429 949
- FR-A- 4 007 674
- US-A- 4 066 743

## Description

### Background of the Invention

This invention provides new nonionic radiographic contrast agents having desirable water solubility and low osmolality properties. These new compounds are derivatives of the 5-amino-2,4,6-triiodo-1,3, benzenecarboxylic acid moiety, wherein the 5 amino nitrogen atom is part of a 4,5 or 6-membered heterocyclic ring.

Ionic contrast agents that contain a heterocyclic ring and an iodobenzene moiety have been reported in the literature. For instance Iodophthalein is disclosed in Amer. J. Pharm., 100, 374 (1928). U.S. 2,776,241 discloses dimeric compounds with heterocyclic bridges. U.S. 3,306,927 discloses heterocycles as counter ions. U.S. 2,750,393 discloses ionic cholecystopaques. British Patent 1,191,015 discloses 3,5-diamino-benzoic acids. U.S. 4,066,743 discloses 5-amino-isophthalic acids. U.S. 4,250,113 discloses 3-aminobenzoic acids.

Non-ionic contrast agents having a heterocyclic ring including sugar ethers, acyl amides or aminosugars and reversed amides from keto-sugars have been disclosed in the prior art.

DE-A-2,726,196 describes the use of 5-N-2-hydroxyethyl (and 2,3-dihydroxypropyl)acetamido-2,4,6-triiodo-N,N'-bis(2, 3-dihydroxypropyl)isophthalamide as non-ionic X-ray contrast agents.

FR-A-2,007,674 describes 2,4,6-triiodo-benzoic acid derivatives having a substituent at the 5-position which is a N-lactam group or a N-lactam-methyl group. Such compounds are used as water-soluble X-ray contrast agents.

Various broad aspects of the present invention are set out in the claims.

The new radiographic contrast agents of this invention have the formula 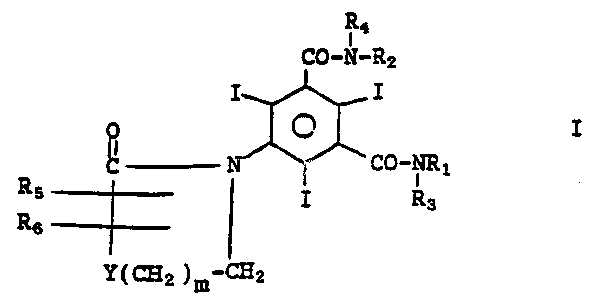
wherein Y is a single bond, -CH₂CH₂-, -CH₂O-, -OCH₂-, -N-CH₂-, 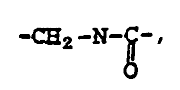
-CH₂N-, -CH₂-, -O- and -N-;
R₁ and R₂ are the same or different and are 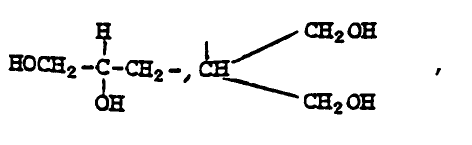
-CH₂CH₂OH 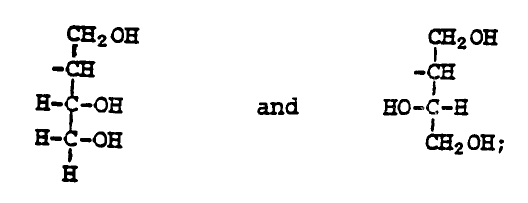
R₃ and R₄ are the same or different and are hydrogen, methyl or -CH₂CH₂OH; R₅ is hydrogen, alkyl, -CH₂CH₂OH, CH₂OH or OH and R₆ is alkyl, -CH₂CH₂OH, CH₂OH, OH or hydrogen and may be the same or different than R₅ and m is zero or one, with the proviso that no methylene or methine carbon atom of the heterocyclic ring is attached to both a nitrogen and an oxygen atom with the additional proviso that when Y is a single bond, m is not zero. The term alkyl refers to straight or branched chain groups of one to six carbon atoms including methyl, ethyl and propyl.

The compounds preferably have a molecular weight of from 780 to 835 and from four to six hydroxy groups per monomeric unit. They preferably have one or two tertiary nitrogen atoms. Compounds with one tertiary atom are most preferred.

Non-ionic contrast agents of the invention have been found to exhibit low toxicity, high chemical stability, ease of chemical synthesis, low viscosity and low osmolality of concentrated aqueous solutions of the contrast agent.

The following groups substituted and unsubstituted are representative of the heterocycles connected to the 5-position of the benzene ring in Formula I: 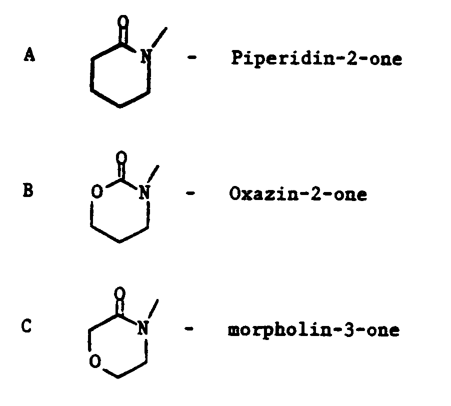 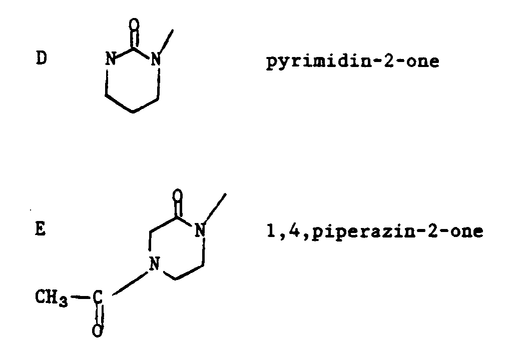 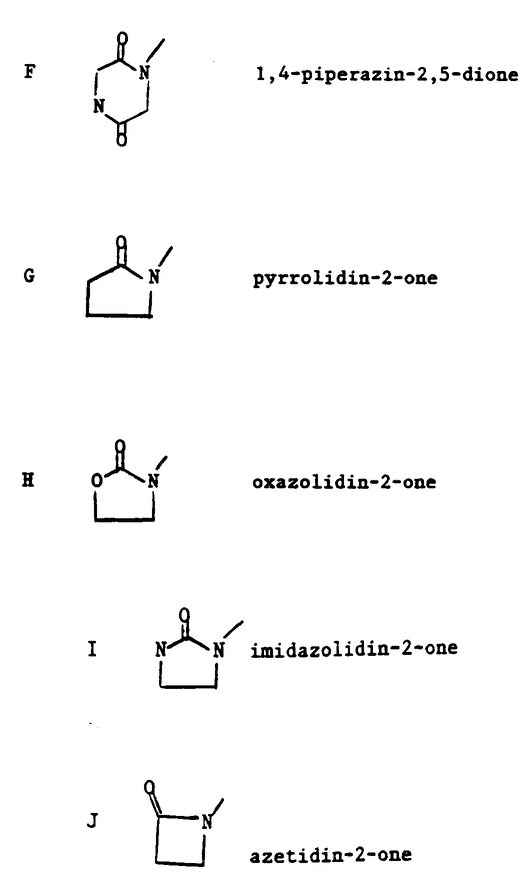
It is noted that when Y is 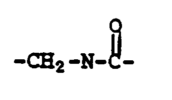
it includes heterocycles represented by formulae E and F.
The pyrrolidin-2-one, morpholin-3-one, piperidin-2-one and oxazolidin-2-one are preferred. Most preferred is hydroxy and hydroxymethyl substitution on the pyrrolidin-2-one ring.

The invention extends to a method of using the compounds as contrast agents for determining conditions within a body cavity. Such determination may be a useful aid in carrying out a diagnosis.

The invention also provides a method of preparing the compounds of the invention which comprises a ring closure reaction of the optionally substituted methylene group onto the nitrogen atom in the heterocyclic ring structure of formula I, the carbon atom of the methylene group being activated for ring closure, preferably by an epoxy group, a halogen atom, or an olefinic bond.

According to another aspect, the invention provides a method of preparing lactams by reacting l-oxo-ω-alkenylamino or 1-oxo-ω-oxaalkenylamino derivatives of 2,4,6-triido 1,3-benzenedicarboxamides with iodinating agents in the presence of a base.

Illustrative preferred preparation methods will now be described by way of example.

The preparation of compounds of formula I wherein the heterocyclic group is oxazolidinyl with 4-hydroxymethyl substitution is illustrated by the following scheme:

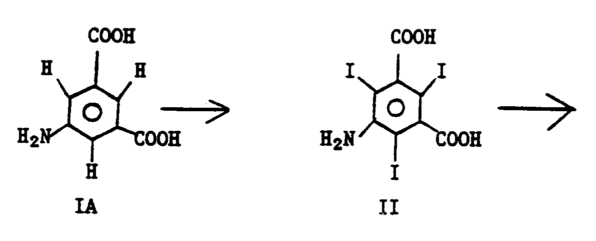

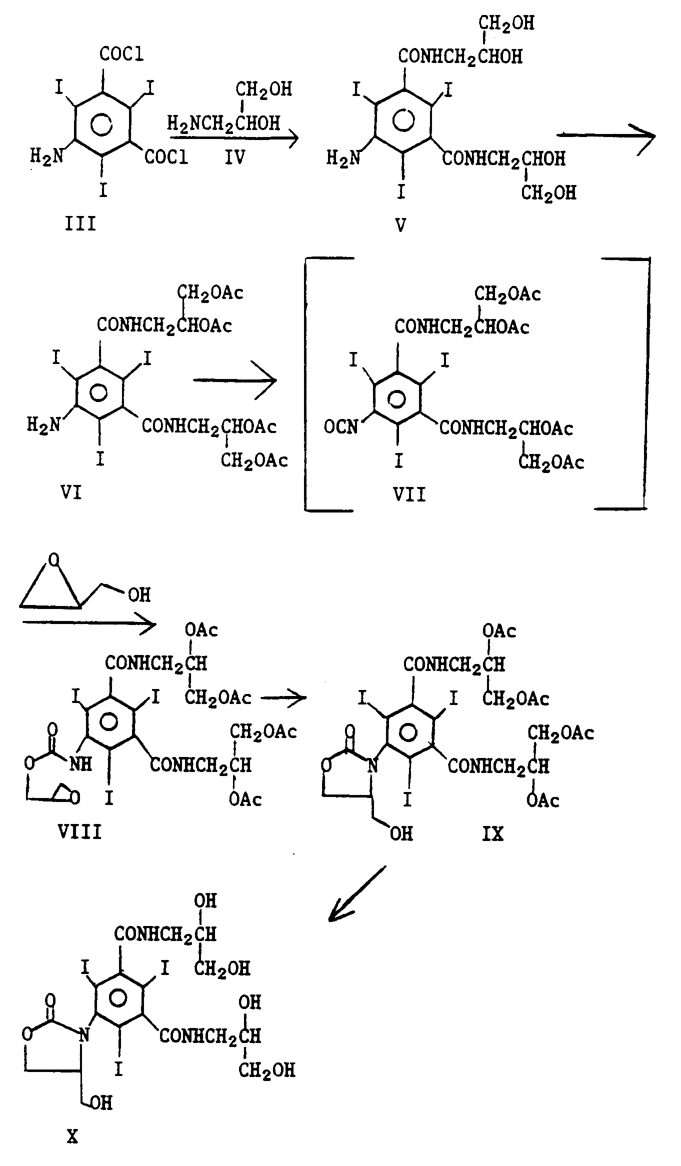

Compound IA which is commercially available is iodinated with a compound such as potassium iododichloride in dilute hydrochloric acid solution to obtain 5-amino-2,4,6,-triiodo-1,3-benzenedicarboxylic acid (II). Compound II is chlorinated with purified thionyl chloride to obtain the corresponding bis-chloride (III). Compound III is then amidated with 1-amino-2,3-propanediol (IV) to obtain the isophthalamide V. Compound V is then selectively O- acylated with acetic anhydride in pyridine to yield 5-amino-N,N′-bis [2,3-bis(acetyloxy)propyl]2,4,6-triiodo-1,3-benzenedicarboxamide (VI). Amino-dehalogenation of compound VI by treatment with a toluene solution of phosgene in ethyl acetate at 60° over a period of sixteen hours results in a conversion into the corresponding isocyanate (VII). When the reaction is over, the solvents along with unreacted excess phosgene and hydrogen chloride, that was liberated during the course of the reaction, are removed by distillation. Any trace of acid, left behind, is removed by repeated co-distillations with ethyl acetate.

Addition of glycidol to the crude isocyanate (VII), in the presence of catalysts such as cuprous chloride or phenylmercuric acetate, in ethyl acetate at room temperature overnight yields oxiranylmethyl [3,5-bis[[[2,3-bis(acetyloxy)-propyl]amino]carbonyl]-2,4,6-triiodophenyl]carbamate (VIII).

A basic solution of the glycidyl carbamate (VIII) is heated at 75° for 30 minutes. Intramolecular cyclization occurs to afford N,N′-bis[2,3-bis(acetyloxy)propyl]-5-[4-hydroxymethyl)-2-oxo-3-oxazolidiny]-2,4,6-triiodo-1,3-benzene-dicarboxamide (IX), as the sole product, after crystallization from aqueous methanol.

Deacetylation of the tetraacetate (IX) by treatment with sodium methoxide in methanol, followed by neutralization with Dowex-50-(H⁺) resin and decolorization with charcoal, yields N,N′-bis(2,3-dihydroxypropyl)-5-[4-(hydroxymethyl)-2-oxo-3-oxazolidinyl]-2,4,6-triiodo-1,3-benzene-dicarboxamide (X). This product is desalted and further purified by low pressure reverse phase column chromatography. Crystallization from water or from aqueous isopropanol yields compound X.

Substituted pyrrolidin-2-one derivatives with no substitution at the 5 position are prepared according to Scheme A. 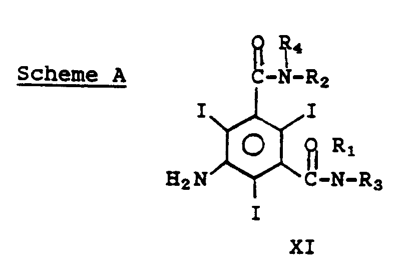 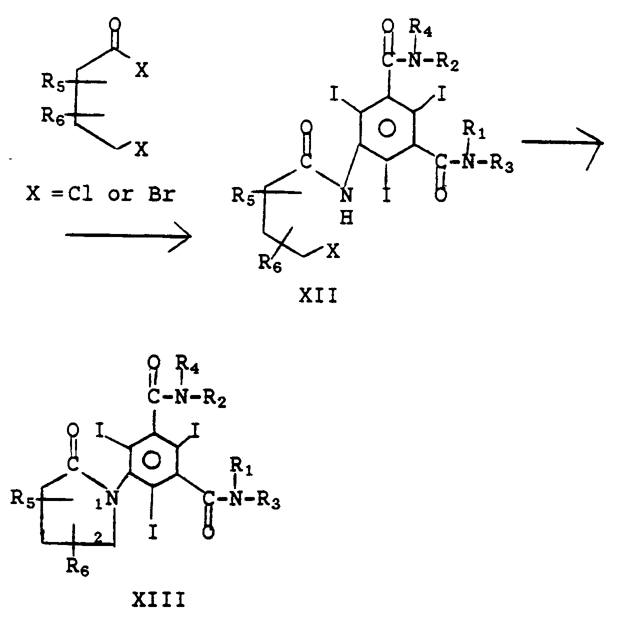
The amide of formula XI is reacted with an ω-halo-acid halide to obtain the anilide of compound XII followed by cyclization of compound XII to the pyrrolidine-2-one of formula XIII.

Alternatively, pyrrolidin-2-one derivatives with a hydroxymethyl substitution at the 5-position are prepared by converting the compound of formula XI into the substituted unsaturated anilide having the formula 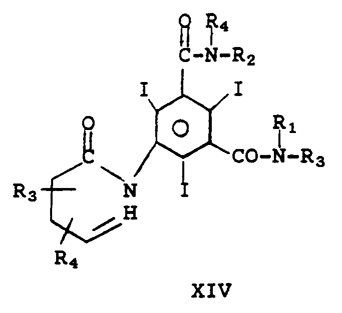
by treatment with a substituted unsaturated acid chloride. Intramolecular cyclization of the compound of formula XIV through a halonium intermediate, by treatment with an iodinating agent such as N-iodosuccinimide under basic conditions provides the corresponding halo-methyl pyrrolidin-2-one having the formula

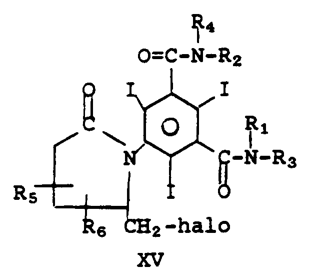

The employment of basic conditions in this cyclization reaction is required to significantly promote N-participative ring closure to afford the pyrrolidine-2-one of formula XV. In the absence of base, O-cyclization predominates affording unwanted 2-imino-tetrahydrofuran derivatives.

If the substituents R₁, R₂, R₃ and R₄ contain acetyloxy groups, they will be deprotected during this ring closure reaction due to the basic conditions employed. The acetyl groups are reintroduced by subjecting the cyclized product to the treatment with acetic anhydride in pyridine.

Compound XV can be converted into the corresponding hydroxy derivative via the acetyloxy derivative by conventional methods known in the art.

The preparation of compounds of formula I wherein the heterocycle group is azetidin-2-one can be accomplished by treating compound XI with the appropriate substituted unsaturated acid chloride to yield the substituted unsaturated anilide represented below: 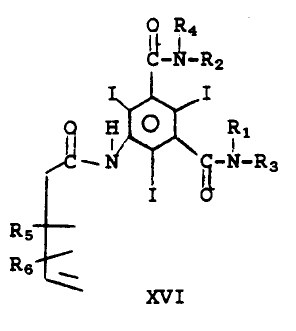
Intramolecular cyclization under basic conditions of compound XVI through a halonium intermediate, by treatment with N-iodosuccinimide provides the corresponding halo compound of the formula: 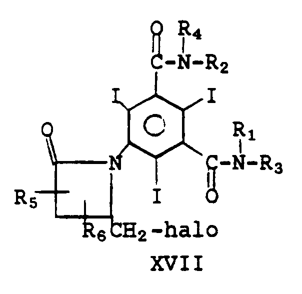
In this cyclization the possibility exists that 3-halopyrrolidinone could result and this forms yet another synthesis of a halo-substituted pyrrolidinone.

Compound XVII can be converted into the corresponding hydroxy derivative via the corresponding acetyloxy compound using conventional methods known in the art. The unsaturated anilide (XVI) can be converted into the corresponding silyl-imidate represented below 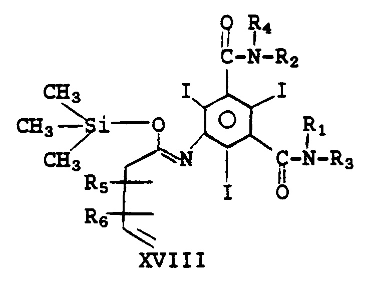
before treatment with iodine or N-iodosuccinimide. Ring closure will then proceed by N-participation as described previously, without competitive O-participation.

The compounds of formula I wherein the heterocyclic group is a piperidin-2-one can be prepared by reacting compound XI with an appropriately substituted bromopentanoyl bromide of the formula 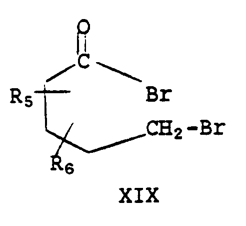
in a suitable solvent such as dimethylacetamide to yield the corresponding anilide of the formula 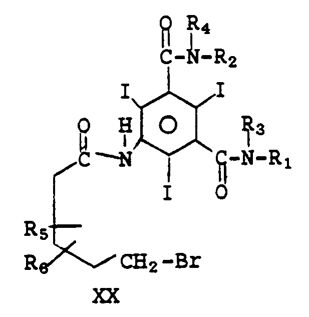
which upon treatment with a base such as potassium carbonate in a suitable solvent such as dimethylacetamide will yield the desired piperidin-2-one of the formula 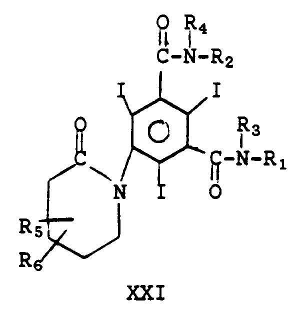
When R₅ or R₆ is hydroxyl or hydroxymethyl in compound XXVI it is protected as the corresponding acetate or ether which is eventually deprotected by conventional means. A halogen substituent on the piperidine-2-one ring can be converted into an acetyloxy group by treatment with silver acetate in acetic acid or with tetraethylammonium acetate in a suitable solvent. Solvolysis of the acetate with aqueous methanolic sodium hydroxide or methanol in the presence of sodium methoxide will give compound XXI wherein R₅ and/or R₆ is hydroxy or CH₂OH. For example, the dibromo-anilide 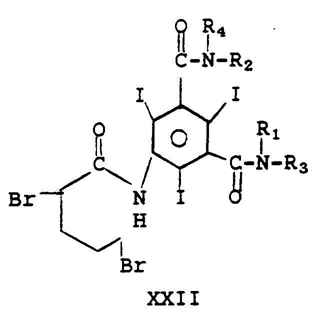
can be ring closed to the 3-bromo-piperidin-2-one 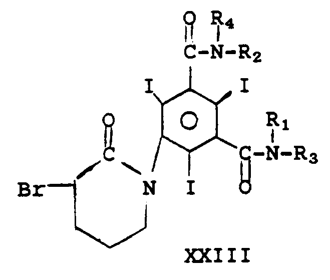
The bromo moiety of compound XXIII can be converted into the acetate and then to the corresponding hydroxy compound by the methods described above.

2,5,dibromopentanoyl bromide used in the preparation of compound XXII is prepared by treating δ-valerolactone with bromine in the presence of red phosphorous. 2,5,dibromopentanoyl bromide is condensed with compound XI to form compound XXII.

The compounds of formula I wherein the heterocyclic group is a morpholin-3-one can be prepared by reacting Compound XI with a β-chloroethoxyacetyl halide such as β-chloroethoxyacetyl chloride in dimethylacetamide to yield the anilide of the formula 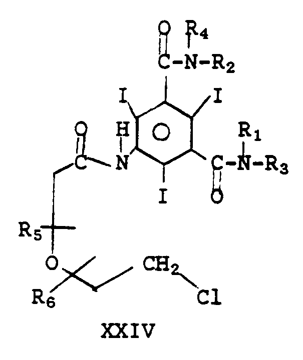
Compound XXIV, under basic conditions will undergo cyclization to provide the morpholin-3-one derivative of the formula

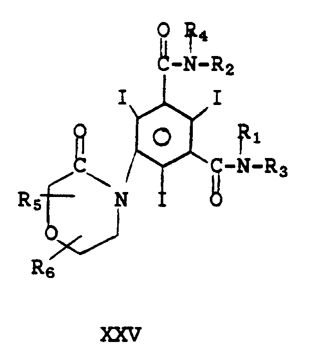

For the preparation of compounds of formula XXV wherein R₅ and R₆ are other than hydrogen, appropriate substituted β-chloroethoxyacetyl halides are used.

Alternatively, condensation of substituted allyloxyacetyl of the formula 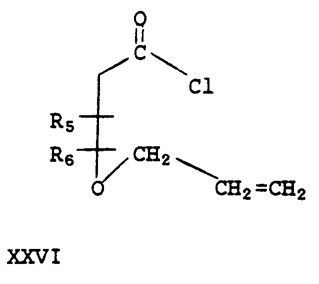
with compound XI will yield the anilide of the formula 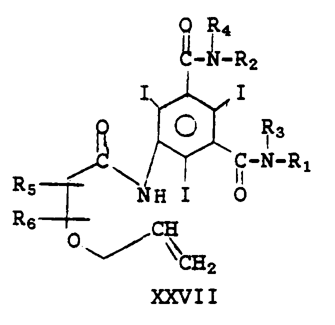
Intramolecular halolactamation under basic conditions by treatment with N-iodosuccinimide yields the corresponding halide of the formula 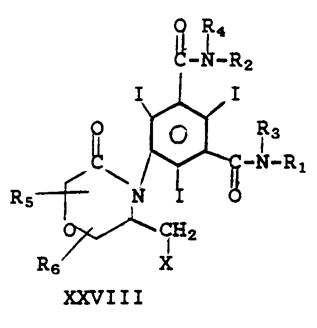
This approach provides morpholin-5-one derivatives with a branched halomethyl substituent at the 3-position. The basic conditions are required to avoid the formulation of undesired O-participative ring closure intermediates. In the presence of sodium methoxide N-ring closure is favored over O-ring closure by a factor of approximately 95:5. If the substituents R₁, R₂, R₃ and R₄ contain acetyloxy groups, they will be deprotected during the ring closure reaction due to the basic conditions employed. The acetyl groups are reintroduced by subjecting the cyclized product to the treatment with acetic anhydride in pyridine.

An alternative way to prevent the amide oxygen of compound XXVII from participating in the above cyclization is to transform the amide function into a silyl derivative according to the procedure of S. Knapp, Tetrahedron Letters, 26, p. 1803 (1985).

Acetolysis of compound XXVII with silver acetate in acetic acid yields the corresponding acetyloxy derivative. Solvolysis of the acetate with sodium methoxide in methanol or with aqueous methanolic sodium hydroxide will yield the hydroxymethyl-morpholinone analog of the formula 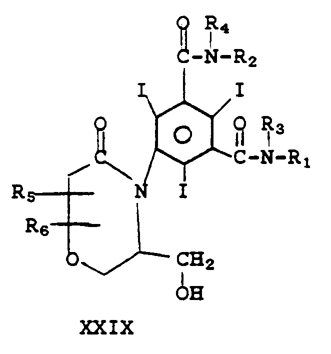
The compounds of formula I wherein the heterocyclic group is a pyrimidin-2-one can be prepared by reacting compound XI with the appropriately substituted haloisocyanate of the formula 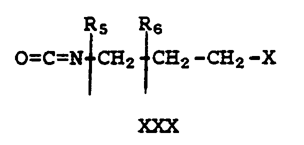
wherein X is chloro or bromo to provide the compound of the following formula 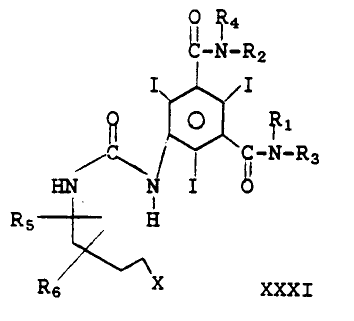
Cyclization in the presence of a base yields the desired pyrimidine 2-one analogs having the formula: 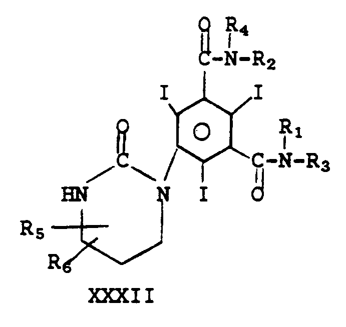
The compounds of formula I wherein the heterocyclic group is a piperazin-2,5-dione can be prepared by reacting compound XI with a compound of the formula 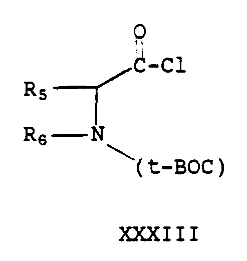
to yield a compound of the formula 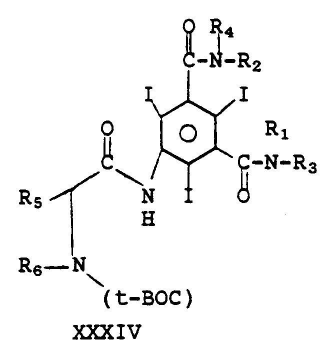
After deprotection of compound XXXIV, the resultant compound can be reacted with chloroacetyl chloride, followed by intramolecular cyclization to yield the desired piperazin-2,5-dione of the formula: 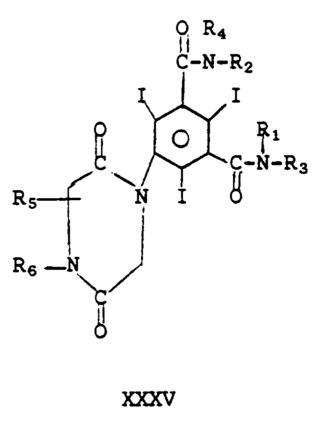
The compounds of formula I wherein the heterocyclic group is oxazin-2-one can be prepared by reacting compound XI with 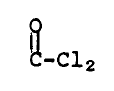
to yield a compound of the formula 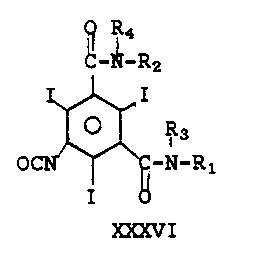
Treatment of compound XXXVI with 1-hydroxy-2-(tetrahydropyron-2-yl)oxy-3-chloropropane in the presence of phenylmercuric acetate yields the carbamate of the formula 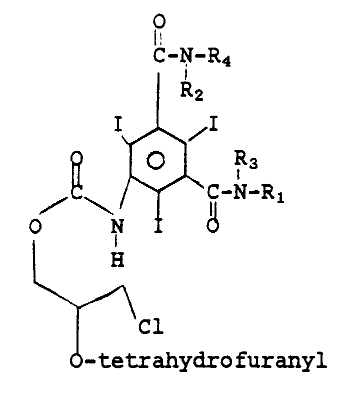
Cyclization of compound XXXVII to the 1,3 oxazin-2-one is accomplished by heating in pyridine or by treatment with sodium hydride in a suitable solvent. The tetrahydropyranyl group is removed by stirring with methanol in the presence of a catalytic amound of p-toluenesulfonic acid to give the desired oxazin-2-one of the formula: 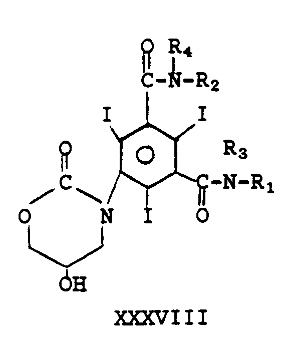
1-hydroxy-2-(tetrahydropyran-2-yl)oxy-3-chloropropane can be prepared by the following scheme: epichlorohydrin is reacted with acetic acid in the presence of a catalytic amount of iron trichloride to give a mixture of 1-acetyloxy-3-chloropropan-2-ol and 2-acetyloxy-3-chloropropan-1-ol, the major component. The mixture is treated with dihydropyran in the presence of p-toluene-sulfonic acid for five hours at 25°C to give a mixture of 1-acetyloxy-2-(tetrahydropyran-2-yl)-oxy-3-chloropropane and 1-(tetrahydropyranyloxy)-2-acetyloxy-3-chloropropane. The mixture is added to a rapidly stirred mixture of aqueous methanol and potassium carbonate at 25°C and stirred for two hours. Evaporation of the methanol, extraction and drying yields a mixture 1-hydroxy-2-(tetrahydropyran-2-yl)oxy-3-chloropropane and 1-(tetrahydropyran-2-yl)oxy-2-hydroxy-3-chloropropane. Fractional vacuum distillation yields pure 1-hydroxy-2-(tetrahydropyran-2-yl)-oxy-3-chloropropane since the minor impurity is converted into the more volatile epoxy compound during the distillation procedure.

Alternatively, oxazinone with a 4-hydroxymethyl substituent can be prepared by reacting compound XXXVI with an appropriately substituted 3,4-epoxybutan-1-ol of the formula 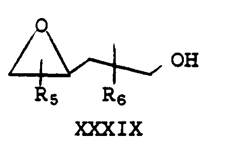
in the presence of phenyl mecuric acetate to yield the carbamate of the formula 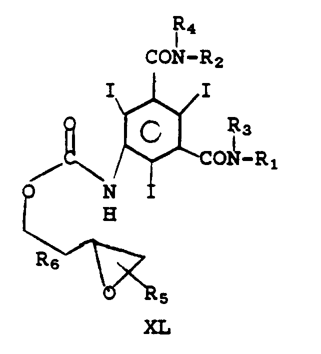
Heating of compound XL in pyridine will yield the corresponding oxazin-2-one of the formula 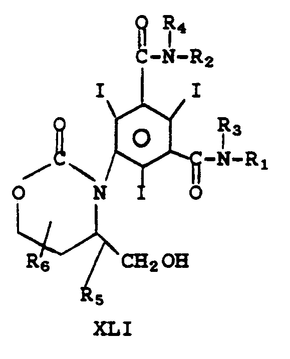
The compound of formula I wherein the heterocycle is imidazolin-2-one can be prepared by reacting compound XXXVI with an allyl amine or an allyl amide in order to obtain the following compound: 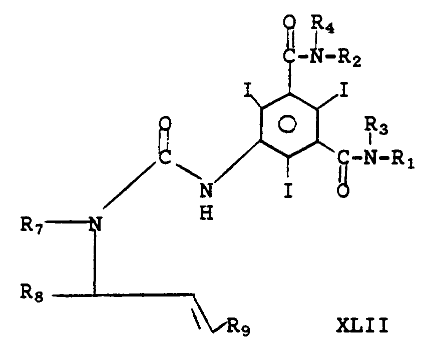
wherein R₇ is hydrogen, lower alkyl or acetyl, R₈ and R₉ are hydrogen or lower alkyl with the provision that only one of R₇, R₈ and R₉ can be lower alkyl. The mixed urea of compound XLII can be silyated with trimethylsilyl triflate as can the mixed acyl-urea 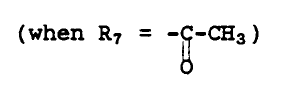
to give the corresponding O-silyated derivatives which upon treatment with N-iodosuccinimide or iodine leads to the formation of the corresponding iodomethylimidazolin-2-ones of the following formulae:

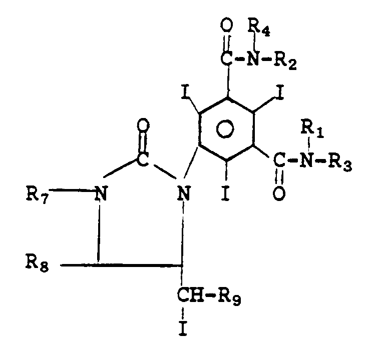

XLIII R₇ = H, CH₃

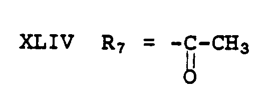

Acetolysis of compound XLIII and XLIV using silver acetate/acetic acid or tetraethylammonium acetate results in the corresponding acetyloxymethyl compounds. Deacetylation will yield the desired compound of formula I, wherein R₇ is H or CH₃. 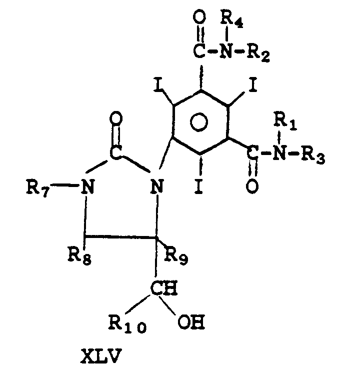
The compound of formula I wherein the heterocycle is piperazine-2-one can be prepared by reacting compound XI with a compound of the formula 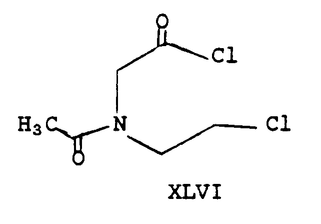
to yield a compound of the following formula 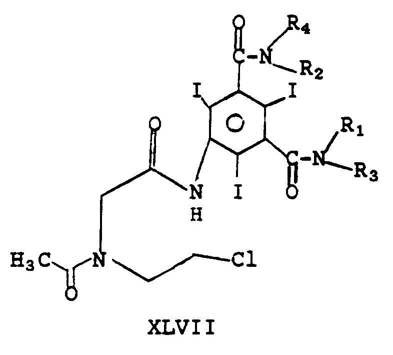
Cyclization in the presence of a base affords the desired piperazine-2-one analogs having the formula 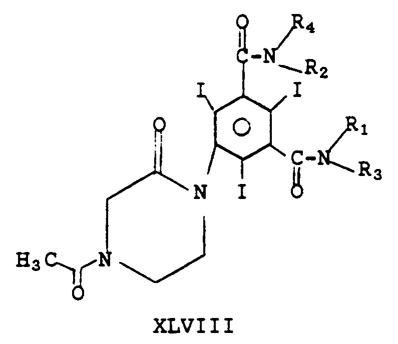
The acyclic synthon of formula XLVI can be made by methods described in prior art and shown in the following scheme 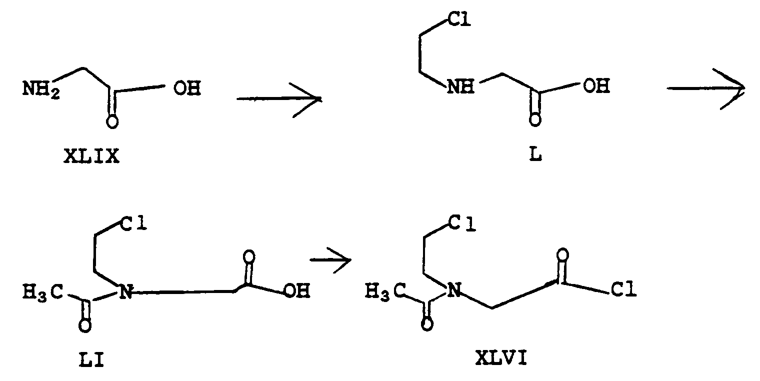
The compounds of the invention are suitable for use in most fields of application in which water soluble radiopaque compounds are necessary, such as vasography, urography, arthrography, and for the visualization of body cavities containing cerebrospinal fluid. When formulated with addition agents which increase the viscosity of the aqueous solutions, they may be employed to advantage for bronchography and hysterosalpingography.

The radio-opaque compounds of the invention are particularly useful as active ingredients of aqueous compositions for visualization of the cardiovascular system and for cerebral angiography. Because of their non-ionic nature, they are suited for visualization of body cavities containing spinocerebral liquor such as in radiculography, ventriculography and myelography.

Aqueous compositions for the applications indicated above may be formulated to contain a single compound of the invention, or more than one compound of the invention, if the individual compounds are very pure.

The radio-opaque compositions of the invention are aqueous solutions containing 15 g and more of the compounds per 100 ml, equivalent to 50 to approximately 500 mg iodine per ml. The more concentrated solutions are generally preferred, and they are applied in a manner generally known and selected according to the body cavity which it it intended to visualize. In vasography, the solutions are injected or infused into the vessels, particularly the blood vessels.
Intravenous injection is resorted to in urography. For myelography and radiculography, the solutions are instilled after lumbar or suoccipital puncture. The amounts of solution necessary generally are 5 to 15 ml for myelography, 3 to 5 ml for radiculography, and 1 to 2 ml in ventriculography.

The X-ray contrast compositions containing the compounds of the invention as active ingredients are prepared in a very simple manner since no salt-forming or solubilizing ingredients are needed. Any one of the compounds of Examples 1 - 6 may be dissolved under sterile conditions in the desired amount of double-distilled water, and the solution so obtained is ready to be received in vials and sterilized. The compounds are not decomposed at sterilizing temperatures during the usual sterilizing periods (30 minutes at 120°C or 60 minutes at 100°C).

The new heterocycle based non-ionic contrast agents described herein have improved features not present in currently available contrast agents. Their superior stability characteristic, eliminates the need to use organic buffers or carbon dioxide saturation during sterilization of their formulations by autoclaving.

The new heterocycle based non-ionic contrast agents described herein are found to have excellent properties as to tolerance, water solubility, stability, osmolality, viscosity and the like, factors important in angiogrophy and urography.

The compound of Example 8 also exhibits anti-coagulant behavior. This property is desirable in a nonionic X-ray contrast agent that is to be used in angiography. Other compounds of formula I may also possess this anti-coagulant behavior.

The following examples are offered by way of illustration and not be way of limitation. All temperatures are given in centrigrade.

### Example 1

### N,N′-Bis(2,3-dihydroxypropyl)-5-[4-(hydroxymethyl)-2-oxo-3-oxazolidinyl]-2,4,6-triiodo-1,3-benzenedicarboxamide

### Example 1a

### [3,5-Bis-[[[2,3-bis(acetyloxy)-propyl]amino]carbonyl]-2,4,6-triiodophenyl]-carbamic acid, oxiranylmethyl ester

To a solution of 5-amino-N,N′-bis[2,3-bis-(acetyloxy)propyl-2,4,6-triiodo-1,3-benzenedicarboxamide, (30.00 g, 0.034 mole) in ethyl acetate (300 ml) was added a toluene solution (2 molar) of phosgene (170 ml, 0.34 mole). The flask was stoppered with a rubber septum and wired tightly. The reaction mixture was stirred at 60°C for 15 hours.

Ethyl acetate and toluene were removed, under vacuum, by slowly raising the temperature to 85 - 90°. After removing the solvents, ethyl acetate (150 ml) was added to the residue and was slowly distilled off. This process was repeated twice. The residue containing the isocyanate product was dried *in vacuo* for 1 h. The crude isocyanate, thus obtained as a colorless solid, was redissolved in ethyl acetate (350 ml) and glycidol (5.4 g, 0.071 mol), followed by phenylmercuric acetate (300 mg), were added to this solution with stirring at room temperature. The reaction mixture was stirred overnight. Undissolved impurities were removed from the reaction mixture by filtration and water (200 ml) was added to the filtrate. The organic layer that separated, was washed with water (2 x 100 ml) and brine (100 ml). It was dried and the solvent was removed, to obtain the glycidyl carbamate, as a nearly colorless solid (32.00 g). To a solution of this solid is ethyl acetate (150 ml), hexane (10 ml) was added and the solution was allowed to stand in the refrigerator overnight. The solid, thus obtained, was filtered and dried, to obtain the glycidyl carbamate, as a crystalline solid (21.6 g). The mother liquor was concentrated and the resulting solid was crystallized, as described above, to obtain an additional 6.3 g of the product. Both the crops were combined and the resulting solid, upon recrystallization once again from a mixture of ethyl acetate (100 ml) and hexane (10 ml), afforded pure oxiranylmethyl [3,5-bis-[[[2,3-bis(acetyloxy)propyl]amino]carbonyl]-2,4,6-triiodophenyl]carbamate, as a white crystalline powder (25.6 g, yield 76.5%), mp, 142-145°.

### Example 1B

### N,N′-Bis[2,3-bis)acetyloxy)propyl]-5-(4-hydroxymethyl)-2-oxo-3-oxazolidinyl]-2,4,6-triiodo-1,3-benzenedicarboxamide

A solution of the oxiranylmethyl ester of Example 1a (30.00 g, 0.030 mole) in freshly distilled anhydrous pyridine (300 ml) was heated at 75°C. The reaction was found to have gone to completion in 2.5 h. Pyridine was removed from the reaction mixture in a rotary evaporator at 50°C and the residue was co-evaporated twice with toluene (100 ml), to remove residual pyridine. The solid, thus obtained, was dissolved in ethyl acetate (100 ml) and, then, precipitated by pouring into toluene (200 ml). The solid was filtered and dried to afford a white powder. The product was crystallized from aqueous methanol. N,N′-bis[2,3-bis(acetyloxy)propyl]-5-[4-hydroxymethyl)-2-oxo-3-oxazolidinyl]-2,4,6-triiodo-1,3-benzenedicarboxamide was obtained as colorless needles. A second crop of 4.6 g was further obtained from the mother liquor. Total yield 75%; mp, 278 - 80°.

### Example 1c

### N,N′-Bis(2,3-dihydroxypropyl)-5-[4-(hydroxymethyl)-2-oxo-3-oxazolidinyl]-2,4,6-triiodo-1,3-benzenedicarboxamide

To a solution of N,N′-Bis[2,3-bis(acetyloxy)-propyl]-5-[4-(hydroxymethyl)-2-oxo-3-oxazolidinyl]-2,4,6-triiodo-1,3-benzenedicarboxamide of Example 1b (18.00 g, 0.0184 mole) in anhydrous methanol (180 ml) was added a methanolic solution of sodium methoxide (1.08 g, 0.02 mole) and the mixture was stirred for 1 h. Dowex-50 (H⁺) resin was added to this solution until the pH was brought down to approximately 7.00. The resin was filtered off, the methanol removed in a rotary evaporator and the resulting syrupy material dissolved in water (150 ml). The solution was decolorized by boiling for 15 minutes with Darco (200 mg). It was then filtered and solvent removal afforded a colorless glass, which was dried in a vacuum oven for 24 hours. The product (14.1 g, yield 95%), thus obtained, had a purity of 99.67%.

The material was further purified by low pressure reverse phase column chromatography, using the CHP-20 resin. The product (4.7 g) was obtained as a white powder. HPLC analysis of this material revealed that only the hydrophobic impurity had been removed by this procedure. The hydrophilic impurity was, however, removed by crystallization from water, affording pure N,N′-bis-(2,3-dihydroxypropyl)-5-[4-(hydroxymethyl)-2-oxo-3-oxazolidinyl]-2,4,6-triiodo-1,3-benzenedicarboxamide, (4.15 g, 99.86%), as fine white needles. mp, 315 - 320° (d.) Recrystallization was also achieved from a mixture of isopropanol and water.

### Example 2

### N,N′-bis(2,3-dihydroxypropyl)-5-[(R)-[4-hydroxymethyl)-2-oxo-3-oxazolidinyl]-2,4,6-triiodo-1,3-benzenedicarboxamide

### Example 2a

### [3,5-Bis-[[[2,3-bis(acetyloxy)propyl]aminocarbonyl[-2,4,6-triiodophenyl]carbamic acid, S-oxiranylmethyl ester

To a solution of 5-amino-N,N′-bis(2,3-bis (acetyloxy)propyl]-2,4,6-triiodo-1,3-benzenedicarboxamide (8.10 g, 0.0093 mol) in ethyl acetate (50 ml) was added a toluene solution (2 molar) of phosgene (Fluka AG) (55 ml, 0.110 mol). The flask was stoppered with a rubber septum and wired tightly. The reaction mixture was stirred at 60°. The isocyanate formation was found to be complete in 18 h. Ethyl acetate and toluene were removed under vacuum at 80°C. After removing the solvents, ethyl acetate (100 ml) was added to the residue and it was slowly distilled off. This process was repeated twice. The distillation assembly was removed and the residue containing the product was dried *in vacuo* for 1 h. The isocyanate obtained as a beige solid, was redissolved in ethyl acetate (350 ml) and, then, treated with S-glycidol (84.6% optically pure by [a]_{D} measurement, 1.4 ml, 0.021 mol) and phenylmercuric acetate (0.145 g). The reaction mixture was stirred for 48 h. at room temperature. Water (100 ml) was added to the light yellow solution, the organic layer separated, and a small amount of insoluble material that was present was filtered off. The organic layer was then washed with water (2 x 350 ml), and brine 1 x 100 ml). It was dried and the solvent removed to obtain the crude carbamate as a beige solid (9.00 g). The solid was dissolved in ethyl acetate (20 ml) and filtered once again. The residue obtained by solvent removal was purified by silica gel column chromatography to obtain [3,5-Bis-[[[2,3-bis(acetyloxy)propyl]aminocarbonyl[-2,4,6-triiodophenyl]carbamic acid, S-oxiranylmethyl ester (5.05 g, 60%) as a colorless glassy solid. m.p. 115 - 118°.

### Example 2b

### N,N′-Bis[2,3-bis(acetyloxy)propyl]-5-[4-(R)-(hydroxymethyl)-2-oxo-3-oxazolidinyl]-2,4,6-triiodo-1,3-benzenedicarboxamide

A solution of the S-oxiranylmethyl ester of Example 2a (2.45 g, 0.0025 mol) in freshly distilled anhydrous pyridine (25 ml) was heated at 60° for 2. h. Pyridine was removed in a rotary evaporator and the residue was co-evaporated with toluene (3 x 20 ml). The resulting orange solid was purified by silica gel column chromatography to obtain N,N′-Bis[2,3-bis(acetyloxy)propyl]-5-[4-(R)-(hydroxymethyl)-2-oxo-3-oxazolidinyl]-2,4,6-triiodo-1,3-benzenedicarboxamide as a colorless glassy solid, (1.68 g, 68.6%).

### Example 2c

### N,N′-Bis[2,3-bis(acetyloxy)propyl]-5-(4)-(R)-(hydroxymethyl)-2-oxo-3-oxazolidinyl]-2,4,6-triiodo-1,3-benzenedicarboxamide

To a solution of N,N′-Bis[2,3-bis(acetyloxy)-propyl]-5-(4)-(R)-(hydroxymethyl)-2-oxo-3-oxazolidinyl]-2,4,6-triiodo-1,3-benzenedicarboxamide of Example 2b (0.410 g, 0.51 mmol) in anhydrous methanol (7 ml) was added a methanolic solution of sodium methoxide (1.18 ml, 1 M solution) and the mixture was stirred at room temperature for 1 h. The methanol was then removed on the rotary evaporator and water (8 ml) was added to redissolve the white residue. Dowex-50 (H⁺) was added to this solution portionwise to bring the pH down to approximately 7.0. The Dowex-50 resin was filtered off, water was removed on the rotary evaporator, and the white solid dried *in vacuo* at 60° over P₂0₅. The solid thus obtained (0.350 g) was redissolved in water (0.5 ml), seeded with a tiny crystal of the racemate and left overnight. The crystallized product was filtered, washed with cold water and dried overnight over P₂O₅ to obtain N,N′-Bis[2,3-bis(acetyloxy)propyl]-5-(4)-(R)-(hydroxymethyl)-2-oxo-3-oxazolidinyl]-2,4,6-triiodo-1,3-benzenedicarboxamide as white crystalline needles (0.32 g, 84%).

### Example 3

### N,N′-Bis[2-hydroxy-1-(hydroxymethyl)ethyl]-5-[4-(hydroxymethyl)-2-oxo-3-oxazolidinyl]-2,4,6-triiodo-1,3-benzenedicarboxamide

### Example 3a

### [3,5-Bis[[[2-(acetyloxy)-1-[(acetyloxy)-methyl]ethyl]amino]carbonyl]-2,4,6-triiodophenyl]carbamic acid, oxiranylmethyl ester

To a solution of the N,N′-bis[2-(acetyloxy)-1-[(acetyloxy)-methyl]ethyl]-5-amino-2,4,6-triiodo-1,3-benzenedicarboxamide (14.4 g, 16.4 mmol) in 1,4-dioxane (150 ml) was added to a toluene solution (2.0 m) of phosgene (124 ml, 248 mmol). The flask was stoppered and wired tightly. This mixture was then heated at 60°C under stirring overnight.

The solvents were removed by slowly raising the temperature to 85 - 90°C *in vacuo*. The solid residue obtained was redissolved in 1,4-dioxane (80 ml) and again freed of the solvent. This process was repeated four times. The distillation assembly was removed and the residue containing the isocyanate intermediate was dried *in vacuo* for 30 minutes.

The isocyanate intermediate, thus obtained as a light yellow colored solid, was redissolved in 1,4-dioxane (125 ml). The dioxane solution was treated with glycidol (3.1 g, 2.7 ml, 41.3 mmol). A catalytic amount of phenylmercuric acetate (170 mg) was added and the reaction mixture stirred at room temperature for 17 h. 1,4-Dioxane was removed at 45° on a rotavapor under diminished pressure. The resulting light yellow solid was dissolved in acetonitrile and the solution was extracted with saturated aqueous sodium chloride (3 x 100 ml). The organic layer was dried, filtered, and the solvent removed to obtain the title compound as a yellow solid (15.7 g).

The crude compound was purified by crystallization from boiling acetonitrile (200 ml) to obtain oxiranylmethyl [3,5-bis[[[2-(acetyloxy)-1-[(acetyloxy)methyl]ethyl]amino]carbonyl]-2,4,6-triiodophenyl]carbamate as an off-white solid. (12.1 g; yield 75%). M.P. 228 - 230°.

### Example 3b

### N,N′-Bis[2-(acetyloxy)-1-[(acetyloxy)methyl]ethyl]-5-[4-(hydroxymethyl)-2-oxo-3-oxazolidinyl]-2,4,6-triiodo-1,3-benzenedicarboxamide

A solution of oxiranylmethyl [3,5-bis[[[2-(acetyloxy)-1-[(acetyloxy)methyl]ethyl]amino]carbonyl]-2,4,6-triiodophenyl]carbamate of Example 3a (12.1 g, 12.4 mmol) in freshly distilled anhydrous pyridine (120 ml) was heated at 75°C for 45 minutes. Pyridine was removed under diminished pressure at 45°C and the residue was co-evaporated twice with toluene (75 ml). The solid thus obtained was dissolved in ethyl acetate (250 ml) and the solution was washed with H₂O (1 x 100 ml). The organic layer was dried (MgSO₄). It was then filtered and the solvent removed to obtain the title compound as a yellow solid (9.2 g).

The crude compound was purified by crystallization from a minimum amount of boiling methanol (30 ml). N,N′-Bis[2-(acetyloxy)-1-[(acetyloxy)methyl]ethyl]-5-[4-(hydroxymethyl)-2-oxo-3-oxazolidinyl]-2,4,6-triiodo-1,3-benzenedicarboxamide was obtained, after filtration and drying, as a white crystalline powder (first crop 4.12 g; second crop 1.45 g). These two crops were combined and recrystallized from methanol once again to obtain the oxazolidin-2-one (4.90 g; yield 40.6%) with a purity of 98.7%, as shown by HPLC, m.p. 235-240°.

Further crops of the product amounting to 3.57 g were obtained form the original mother liquor. TLC indicated an approximate purity of 95%, the impurities consisting of two more polar compounds.

### Example 3c

### N,N′-Bis[2-Hydroxy-1-(hydroxymethyl)ethyl]-5-[4-hydroxymethyl)-2-oxo-3-oxazolidinyl]-2,4,6-triiodo-1,3-benzenedicarboxamide

To a solution of N,N′-Bis[2-(acetyloxy)-1-[acetyloxy)methyl]ethyl]-5-[4-(hydroxymethyl)-2-oxo-3-oxazolidinyl]-1,3-benzenedicarboxamide of example 3b (4.2 g, 4.3 mmol) in anhydrous methanol (55 ml) was added a 1 M solution of sodium methoxide in methanol (10 ml) at 0°C. The slurry was stirred at room temperature for 1 h. TLC analysis of the reaction mixture revealed that the deacetylation was complete. Dowex-50-(H⁺) resin was added to the reaction mixture until the pH was brought down to 7. The resin was filtered and the methanol removed in a rotavapor. The resulting solid reside (3.43 g) was dissolved in H₂O (250 ml) and the solution decolorized by boiling for 15 minutes with darco (200 mg). It was then filtered and removal of the solvent afforded a colorless glass which was dried in a vacuum oven for 24 h. The product (3.2 g; yield 92%), thus obtained, had a purity of 99.69%, as determined by HPLC.

This product was further purified by low pressure reverse phase chromatography over CHP-20P resin to obtain the pure title compound N,N′-bis-[2-hydroxy-1-(hydroxymethyl)ethyl]-5-[4-hydroxymethyl)-2-oxo-3-oxazolidinyl]-2,4,6-triiodo-1,3-benzenedicarboxamide (6.2 g, 90% recovery), as a snow-white, glassy solid. HPLC analysis revealed that this sample had negligible amounts of any detectable impurities.

The product was further purified by crystallization from aqueous isopropanol and obtained as colorless clusters of needles.

### Example 4

### N,N′-Bis-[2-hydroxy-1-(hydroxymethyl)ethyl]-5-[3-hydroxy-2-oxo-1-pyrrolidinyl]-2,4,6-triiodo-1,3-benzenedicarboxamide

### Example 4a

### N,N′-Bis-[2-acetyloxy-1-(acetyloxymethyl)ethyl]-5-[3-bromo-2-oxo-1-pyrrolidinyl]-2,4,6-triiodo-1,3-benzenedicarboxamide

A solution of N,N′-bis-[2-acetyloxy-1-(acetyloxymethyl)ethyl]-5-[amino]-2,4,6-triiodo-1,3-benzenedicarboxamide, (8.73 g, 10 mmol) in dry N,N-dimethylacetamide (100 ml) was treated with 2,4-dibromobutyroyl bromide (4.017 g, 13 mmol) under nitrogen and the mixture was stirred for 13 hours at 25°C. The dimethylacetamide was removed by distillation at 55-60°C under high vacuum. The resulting thick paste was dissolved in dry N,N-dimethylacetamide (160 ml) and potassium carbonate (1.80 g, 13 mmol) was added. The mixture was stirred for 60 minutes. An additional portion of potassium carbonate (1.80 g, 13.0 mmol) was added and the mixture stirred for 80 minutes. The suspended salts were filtered off and the volume of the reaction mixture was reduced to about 80 ml by vacuum distillation at 0.05-0.10 mm/Hg. The residual solution was poured slowly with rapid stirring into ice-water (900 ml). The resulting mixture was stirred overnight at 0°C and then filtered. The collected precipitate was dried in a vacuum desiccator (P₂O₅) and then crystallized from acetonitrile to give an off-white powder. This product was treated with acetic anhydride (30 ml) and pyridine (42 ml) for 32 hours at 25°, after which the volatile components were completely removed under high vacuum to obtain N,N′-bis-[2-acetyloxy-1-(acetyloxymethyl)ethyl]-5-[3-bromo-2-oxo-1-pyrrolidinyl]-2,4,6-triiodo-1,3-benzenedicarboxamide as a white powder (4.67 g, 45% yield), m.p. 232-234°.

### Example 4b

### N,N′-Bis-[2-acetyloxy-1-(acetyloxymethyl)ethyl]-5-[3-acetyloxy-2-oxo-1-pyrrolidinyl]-2,4,6-triiodo-1,3-benzenedicarboxamide

N,N′-bis-[2-acetyloxy-1-)acetyloxymethyl)ethyl]-5-[3-bromo-2-oxo-1-pyrrolidinyl]-2,4,6-triiodo-1,3-benzenedicarboxamide (4.00 g, 3.92 mmol) of example 4a was dissolved in glacial acetic acid (40 ml) and treated with silver acetate (3.25 g, 19.50 mmol) at 133-135° for 26 hours under a nitrogen atmosphere. The suspended solids were filtered off and the solvent was removed in vacuo. The resulting crude residue was treated with acetic anhydride (30 ml) and pyridine (42 ml), after which the volatile components were completely removed under high vacuum. The resulting product was purified by silica gel flash chromatography to obtain N,N′-bis-[2-acetyloxy-1-(acetyloxymethyl)ethyl]-5-[3-acetyloxy-2-oxo-1-pyrrolidinyl]-2,4,6-triiodo-1,3-benzenedicarboxamide as a pale orange foam (3.20 g, 82% yield).

### Example 4c

### N,N′-Bis-[2-hydroxy-1-(hydroxymethyl)ethyl[-5-[3-hydroxy-2-oxo-1-pyrrolidinyl]-2,4,6-triiodo-1,3-benzenedicarboxamide

N,N′-Bis-[2-acetyloxy-1-(acetyloxymethyl)-ethyl]-5-[3-acetyloxy-2-oxo-1-pyrrolidinyl]-2,4,6-triiodo-1,3-benzenedicarboxamide of example 4b (2.78 g, 2.78 mmol) was treated with a solution of sodium methoxide in methanol, prepared by the dissolution of sodium (0.032 g, 1.39 mmol) in dry methanol (13 ml), for 4 hours at 25° under an atmosphere of nitrogen to obtain a single major product. The mixture was neutralized to pH 6.98 using Dowex-50 resin (H⁺ form). Filtration of the mixture and evaporation of the solvent gave an orange foamy product (1.89 g), which was purified by low pressure reverse phase column chromatography over CHP-20 resin to obtain N,N′-Bis-[2-hydroxy-1-(hydroxymethyl)ethyl]-5-[3-hydroxy-2-oxo-1-pyrrolidinyl]-2,4,6-triiodo-1,3-benzenedicarboxamide as a white foam (1.54 g, 70% yield).

### Example 5

### N,N′-Bis-[2,3-dihydroxypropyl]-5-[3-hydroxy-2-oxo-1-pyrrolidinyl]-2,4,6-triiodo-1,3-benzenedicarboxamide

### Example 5a

### N,N′-Bis-[2,3-diacetyloxypropyl]-5-[3-bromo-2-oxo-1-pyrrolidinyl]-2,4,6-triiodo-1,3-benzenedicarboxamide

A solution of N,N′-bis-[2,3-diacetyloxypropyl]-5-amino-2,4,6-triiodo-1,3-benzenedicarboxamide (8.73 g, 10 mmol) in N,N-dimethylacetamide (100 ml) was treated with 2,4-dibromobutyroyl bromide (4.02 g, 13.0 mmol) under an atmosphere of nitrogen and the reaction mixture was stirred for 50 hours at ambient temperature. The mixture was then treated with ground potassium carbonate (1.65 g, 12.0 mmol) and stirred for 30 minutes at ambient temperature. An additional portion (1.65 g, 12.0 mmol) of finely ground potassium carbonate was added and the mixture was stirred for 1.9 hours. The suspended salts were filtered and the solvent was evaporated to obtain a brown oil. This crude product was purified by silica gel flash chromatography to isolate N,N′-Bis-[2,3-diacetyloxypropyl]-5-[3-bromo-2-oxo-1-pyrrolidinyl]-2,4,6-triiodo-1,3-benzenedicarboxamide, as a white powder (7.28 g, 71% yield), m.p. 110-112°.

### Example 5b

### N,N′-Bis-[2,3-diacetyloxypropyl]-5-[3-acetyloxy-2-oxo-1-pyrrolidinyl]-2,4,6-triiodo-1,3-benzenedicarboxamide

N,N′-Bis-[2,3-diacetyloxypropyl]-5-[3-bromo-2-oxo-1-pyrrolidinyl]-2,4,6-triiodo-1,3-benzenedicarboxamide of example 5a, (6.43 g, 6.30 mmol) was dissolved in glacial acetic acid (63 ml) and then treated with silver acetate (4.21 g, 25.21 mmol) at reflux under nitrogen for 26 hours. The reaction mixture was cooled to room temperature and the solids filtered off. The solvent was evaporated in vacuo and the residue was partitioned between ethyl acetate and brine. The organic layer was dried and removal of the solvent afforded the crude product as a colored foam. Purification by silica gel flash chromatography furnished N,N′-bis-[2,3-diacetyloxypropyl]-5-[3-acetyloxy-2-oxo-1-pyrrolidinyl]-2,4,6-triiodo-1,3-benzenedicarboxamide as an off white foam (4.45 g, 71% yield).

### Example 5c

### N,N′-Bis-[2,3-dihydroxypropyl]-5-[3-hydroxy-2-oxo-1-pyrrolidinyl]-2,4,6-triiodo-1,3-benzenedicarboxamide

N,N′-Bis-[2,3-diacetyloxypropyl]-5-[3-acetyloxy-2-oxo-1-pyrrolidinyl]-2,4,6-triiodo-1,3-benzenedicarboxamide of example 5b (4.20 g, 4.20 mmol) was treated with a 0.105 M solution of methanolic sodium methoxide [prepared by the addition of Na metal (48 mg, 2.10 mmol) to dry methanol (20 ml)] under a nitrogen atmosphere for 2.5 hours. The pH was then adjusted to 6.70 using Dowex-50 resin (H⁺ form) and AG-1 (OH⁻ form) as necessary. The resin was removed and the solvents evaporated to give a yellow oil. This product was dissolved in 20 ml of deionized water and the pH adjusted to 6.98 by the addition of AG-1 (OH⁻1 form) resin. The resin was filtered off and the filtrate containing the crude product was purified by low pressure reverse phase column chromatography using a CHP-20 resin to obtain N,N′-bis-[2,3-dihydroxypropyl]-5-[3-hydroxy-2-oxo-1-pyrrolidinyl]-2,4,6-triiodo-1,3-benzenedicarboxamide as a white foam (2.36 g, 71% yield).

### Example 6

### 5-[2-(hydroxymethyl)-5-oxo-1-pyrrolidinyl]-N,N′-bis(2,3-dihydroxypropyl]-2,4,6-triiodo-1,3-benzenedicarboxamide

### Example 6a

### N,N′-Bis[2,3-bis(acetyloxy)propyl]-2,4,6-triiodo-5-[(1-oxo-4-pentenoyl)amino]-1,3-benzenedicarboxamide

4-Pentenoyl chloride (7.44 g, 62 mmol) was added to a stirred solution of 5-amino-N,N′-bis-[2,3-bis(acetyloxy)propyl]-2,4,6-triiodo-1,3-benzenedicarboxamide (21.8 g, 25 mmol) in dimethylacetamide (150 ml) at room temperature and the mixture was stirred for 16 hours. Dimethylacetamide was removed in vacuo and the residue dissolved in ethyl acetate (250 ml). The solution was washed with aqueous sodium bicarbonate (10%, 100 ml) and water (2 x 100 ml). The organic layer was dried and the solvent removed to obtain the crude product as an off-white solid. Purification by crystallization from a mixture of ethyl acetate (250 ml) and hexane (50 ml) afforded N,N′-bis[2,3-bis(acetyloxy)propyl]-2,4,6-triiodo-5-[(1-oxo-4-pentenoyl)amino]-1,3-benzenedicarboxamide as a fine powder (21.3 g, yield 89%).

### Example 6b

### N,N′-Bis[2,3-bis(acetyloxy)propyl]-5-[2-(iodomethyl)-5-oxo-1-pyrrolidinyl]-2,4,6-triiodo-1,3-benzenedicarboxamide

To a solution of N,N′-bis[2,3-bis(acetyloxy)propyl]-2,4,6-triiodo-5-[(1-oxo-4-pentenoyl)amino]-1,3-benzenedicarboxamide of example 6a (19.1 g, 20 mmol) in methanol (200 ml) was added a solution of sodium methoxide in methanol, prepared by dissolving sodium (1.38 g, 60 mmol) in methanol (30 ml). The mixture was stirred for 1 hour. The solvent was removed in vacuo and the residue was dissolved in a mixture of methanol and water (1:1, v/v; 200 ml). N-Iodosuccinimide (13.3 g, 60 mmol) was added and the mixture stirred at room temperature for 48 hours. The solvents were distilled off and the residue azeotroped with pyridine (3 x 100 ml). The residue was dissolved in pyridine (150 ml) and treated with acetic anhydride (20.4 g, 200 mmol) with stirring for 17 hours at room temperature. The excess of pyridine and acetic anhydride were removed in vacuo, the residue dissolved in ethyl acetate (250 ml), and the resulting solution washed successively with water (200 ml), aqueous sodium thiosulfate (25%, 2 x 125 ml), and water (2 x 150 ml). The organic layer was dried and removal of the solvent afforded the product as a light yellow glassy solid (20.2 g). The crude product was purified by column chromatography over silica gel to obtain N,N′-bis[2,3-bis(acetyloxy)propyl]-5-[2-(iodomethyl)-5-oxo-1-pyrrolidinyl]-2,4,6-triiodo-1,3-benzenedicarboxamide as a glassy solid (13.8 g, yield 64%).

### Example 6c

### 5-[2-[(Acetyloxy)methyl-5-oxo-1-pyrrolidinyl]-N,N′-bis(acetyloxy)propyl]-2,4,6-triiodo-1,3-benzenedicarboxamide

To a solution of N,N′-bis[2,3-bis(acetyloxy)propyl]-5-[2-(iodomethyl)-5-oxo-1-pyrrolidinyl]-2,4,6-triiodo-1,3-benzenedicarboxamide of example 6b (13.2 g, 150 mmol) in glacial acetic acid (165 ml) was added silver acetate (6.00 g, 35 mmol), and the mixture stirred at 100°C for 16 hours. The insoluble materials were filtered off, acetic acid removed in vacuo at 60°, and the residue dissolved in a mixture of ethyl acetate (200 ml) and water (100 ml). The ethyl acetate layer was dried and removal of the solvent afforded the crude product as a light pink colored solid. This material was purified by column chromatography over silica gel to obtain 5-[2-[(acetyloxy)methyl-5-oxo-1-pyrrolidinyl]-N,N′-bis(acetyloxy)propyl]-2,4,6-triiodo-1,3-benzenedicarboxamide as a light pink glassy solid (8.5 g, yield 79%).

### Example 6d

### 5-[2-(Hydroxymethyl)-5-oxo-1-pyrrolidinyl]-N,N′-bis(2,3-dihydroxypropyl]-2,4,6-triiodo-1,3-benzenedicarboxamide

A solution of 5-[2-[(acetyloxy)methyl-5-oxo-1-pyrrolidinyl N,N′-bis(acetyloxy)propyl]-2,4,6-triiodo-1,3-benzenedicarboxamide of example 6c (5.06 g, 5 mmol) in methanol (50 ml) was treated with a solution of sodium methoxide in methanol, prepared from sodium (115 mg, 5 mmol) and methanol (5 ml). The solution was stirred at room temperature for 1 hour. The pH of the solution was then adjusted to 7 and the solvent removed in vacuo to obtain the crude product. Purification by low pressure reverse phase column chromatography using the CHP-20 resin afforded 5-[2-(hydroxymethyl)-5-oxo-1-pyrrolidinyl]-[N,N′-bis(2,3-dihydroxypropyl]-2,4,6-triiodo-1,3-benzenedicarboxamide as a colorless solid (3.2 g, yield 83%).

This glassy product was crystallized from n-butanol to obtain the title compound as a microcrystalline white powder.

### Example 7

### N,N′-Bis[2-Hydroxy-1-(hydroxymethyl)ethyl]-5-[3-(hydroxymethyl)-5-oxo-4-morpholinyl]-2,4,6-triiodo-1,3-benzenedicarboxamide

### Example 7a

### N,N′-Bis[2-(acetyloxy)-1-[(acetyloxy)methyl]ethyl]-5-[[(2-propenyloxy)acetyl]amino]-2,4,6-triiodo-1,3-benzene-dicarboxamide

Allyloxyacetyl chloride (9.0 g, 67 mmol) was added dropwise to a stirred solution of N,N′-bis[2-(acetyloxy)-1-[(acetyloxy)methyl]ethyl]-5-amino-2,4,6-triiodo-1,3-benzenedicarboxamide (30.0 g, 34 mmol) in N,N-dimethylacetamide (70 ml) at 0-5°C. The reaction mixture was stirred at 5°C for 30 minutes and at room temperature for 20 hours. It was then slowly added dropwise to a well stirred mixture of ice-water (1.5 L), when a white solid separated out. This was collected by filtration, washed with water, and dried in vacuo to obtain N,N′-Bis[2-(acetyloxy)-1-[(acetyloxy)methyl]ethyl]-5-[(2-propenyloxy)acetyl]amino-2,4,6-triiodo-1,3-benzenedicarboxamide as a white amorphous solid (32.1 g, 97% yield), m.p. 214-16°.

### Example 7b

### N,N′-Bis[2-(acetyloxy)-1-[(acetyloxy)methyl]-2,4,6-triiodo-5-[3-(iodomethyl)-5-oxo-4-morpholinyl]-1,3-benzene-dicarboxamide

To a solution of N,N′-bis[2-(acetyloxy)-1-[(acetyloxy)methyl]ethyl]-5-[(2-propenyloxy)acetyl]amino-2,4,6-triiodo-1,3-benzenedicarboxamide of example 7a (1.95 g, 2 mmol) in dioxane (20 ml) and methanol (10 ml), was added aqueous sodium hydroxide (1 M) 15 ml, 15 mmol). After stirring for 2 hours, N-iodosuccinimide (0.45 g, 2 mmol) was added in portions over a 1 hour period. After 2 hours, more N-iodosuccinimide (0.45 g, 2 mmol) was added in portions to the clear yellow solution, and the stirring continued for 40 hours. The pH of the solution was adjusted to 7 and the solvent removed in vacuo at 40°C. A solution of the residue, thus obtained, in a mixture of pyridine (10 ml) and acetonitrile (5 ml) was treated with acetic anhydride (10 ml) and the reaction mixture stirred for 24 hours at room temperature. The solvents were completely removed in vacuo and the brown residue, upon chromatography over silica gel furnished pure N,N′-Bis[2-(acetyloxy)-1-[(acetyloxy)methyl]ethyl]-2,4,6-triiodo-5-[3-(iodomethyl)-5-oxo-4-morpholinyl]-1,3-benzene-dicarboxamide as a white fluffy solid (1.38 g, 63% yield).

### Example 7c

### N,N′-Bis[2-(acetyloxy)-1-[(acetyloxy)methyl]ethyl]-5-[3-[(acetyloxy)methyl]-5-oxo-4-morpholinyl]-2,4,6-triiodo-1,3-benzenedicarboxamide

To a solution of N,N′-Bis[2-(acetyloxy)-1-[(acetyloxy)methyl]ethyl]-2,4,6-triiodo-5-[3-(iodomethyl)-5-oxo-4-morpholinyl]-1,3-benzenedicarboxamide of example 7b (2.01 g, 1.83 mmol) in glacial acetic acid (35 ml), was added silver acetate (0.67 g, 4 mmol) and the mixture refluxed for 14 hours. The solvent was removed in vacuo at 40°, and the residue extracted with ethyl acetate (200 ml). The organic extract, after washing with saturated aqueous sodium bicarbonate (3 x 25 ml) and water (3 x 25 ml), was dried over anhydrous sodium sulfate. Removal of the solvent followed by purification of the crude product by column chromatography over silica gel, yielded analytically pure N,N′-Bis[2-(acetyloxy)-1-[(acetyloxy)methyl]ethyl]-5-[3-[(acetyloxy)methyl]-5-oxo-4-morpholinyl]-2,4,6-triiodo-1,3-benzenedicarboxamide as a white fluffy solid (1.5 g, 82% yield), m.p. 208-210°.

### Example 7d

### N,N′-Bis[2-Hydroxy-1-(hydroxymethyl)ethyl]-5-[3-(hydroxymethyl)-5-oxo-4-morpholinyl]-2,4,6-triiodo-1,3-benzenedicarboxamide

N,N′-Bis[2-(acetyloxy-1-[(acetyloxy)methyl]ethyl]-5-[3-[(acetyloxy)methyl]-5-oxo-4-morpholinyl]-2,4,6-triiodo-1,3-benzenedicarboxamide of example 7c (1.03 g, 1 mmol) was added to a solution of sodium methoxide in methanol, prepared from sodium (23 mg, 1 mmol) and anhydrous methanol (20 ml). The solution was stirred for 4 hours at room temperature. The pH of the solution was then adjusted to 7 by the addition of Dowex-50 (H⁺) resin. The resin was filtered off and the solvent removed from the filtrate to obtain a white solid (0.8 g) which, upon purification by low pressure reverse phase column chromatography over the CHP-20P resin yielded pure N,N′-Bis[2-Hydroxy-1-(hydroxymethyl)ethyl]-5-[3-(hydroxymethyl)-5-oxo-4-morpholinyl]-2,4,6-triiodo-1,3-benzenedicarboxamide as a snow-white glassy solid (0.65 g, 79% yield).

### Example 8

### N,N′-Bis[2,3-dihydroxypropyl)-5-(3-hydroxy-2-oxo-1-piperidinyl)-2,4,6-triiodo-1,3-benzenedicarboxamide

### Example 8a

### N,N′-Bis[2,3-bis(acetyloxy)propyl]-5-[(2,5-dibromo-1-oxopentyl)amino]-2,4,6-triiodo-1,3-benzenedicarboxamide

To a mixture of γ-valerolactone (20 g, 18.5 ml, 0.2 mol) and red phosphorus (2.31 g, 74.6 mmol) was added at 0°C bromine (10.8 ml, 0.21 mol) dropwise with stirring over a half hour period in an atmosphere of nitrogen. The bath temperature was raised to 70° and more bromine (10.8 ml, 0.21 mol) was added dropwise over a half hour period. The solution was then heated at 80°C for 3.0 hours. Dry nitrogen gas was bubbled into the cooled reaction mixture for one hour to remove the hydrogen bromide generated and the excess of bromine. The light red reaction mixture was distilled under reduced pressure (74-78°, 0.15mm Hg) to obtain the crude product as a slightly colored oil (43.8 g). Fractional distillation of the crude product, furnished pure 2,5-dibromopentanoyl bromide as a colorless oil (35.3 g, yield 55%), b.p. 64-66°/ 0.1 mm/Hg.

2,5-Dibromopentanoyl bromide (11 g, 34 mmol) was added dropwise to a stirred solution of N,N′-bis[2,3-bis(acetyloxy)propyl]-5-amino-2,4,6-triiodo-1,3-benzenedicarboxamide (23.0 g, 26.3 mmol) in N,N-dimethylacetamide (240 ml) at 0°. After the addition, the reaction mixture was stirred at 0° for 1 hour, and then at room temperature for 20 hours. The solvent was removed in vacuo at 45° and the resulting solid was dissolved in ethyl acetate (400 ml). The organic layer was washed with saturated aqueous sodium bicarbonate (1 x 70 ml), water (1 x 70 ml) and saturated sodium chloride (1 x 70 ml). After drying over magnesium sulfate, the solvent was removed in vacuo to obtain a yellow residue (28 g). Purification by column chromatography over silica gel furnished pure N,N′-bis[2,3-bis-(acetyloxy)propyl]-5-[(2,5-dibromo-1-oxopentyl)amino]-2,4,6-triiodo-1,3-benzenedicarboxamide as an off-white crystalline compound (20.2 g, yield 69%).

### Example 8b

### N,N′-Bis[2,3-bis(acetyloxy)propyl]-5-(3-bromo-2-oxo-1-piperidinyl]-2,4,6-triiodo-1,3-benzenedicarboxamide

To a solution of N,N′-bis[2,3-bis(acetyloxy)propyl]-5-[(2,5-dibromo-1-oxopentyl)amino]-2,4,6-triiodo-1,3-benzenedicarboxamide of example 8a (20.7 g, 18.5 mmol) in N,N-dimethylacetamide (200 ml), was added powdered potassium carbonate (20 g, 92 mmol) and the mixture was stirred at room temperature for 5 hours. The resulting slurry was filtered and the filtrate freed of the solvent to obtain a light brown solid, which was redissolved in ethyl acetate (500 ml). The solution was washed with water (2 x 50 ml) and saturated aqueous sodium chloride (1 x 100 ml) and then dried over magnesium sulfate. The solvent was removed in vacuo and the crude product (18.1 g) purified by column chromatography over silica gel to obtain pure N,N′-bis[2,3-bis(acetyloxy)propyl]-5-(3-bromo-2-oxo-1-piperidinyl]-2,4,6-triiodo-1,3-benzenedicarboxamide as a white solid (14.04 g, 80% yield), m.p. 232-35°.

### Example 8c

### 5-[3-(Acetyloxy)-2-oxo-1-piperidinyl]N,N′-bis[2,3-bis(acetyloxy)propyl]-2,4,6-triiodo-1,3-benzenedicarboxamide

To a solution of N,N′-bis[2,3-bis(acetyloxy)-propyl]-5-(3-bromo-2-oxo-1-piperidinyl)-2,4,6-triiodo-1,3-benzenedicarboxamide of example 8b (13.7 g, 13.3 mmol) in glacial acetic acid (400 ml), was added silver acetate (5.6 g, 33.5 mmol) and the mixture was refluxed for 21 hours. After cooling to room temperature, the mixture was filtered, and the filtrate concentrated in vacuo. The residue thus obtained, was redissolved in ethyl acetate (500 ml) and washed successively with water (50 ml), saturated aqueous sodium bicarbonate (3 x 50 ml and saturated aqueous sodium chloride (50 ml). After drying over magnesium sulfate, the solvent was evaporated in vacuo and the crude product, that resulted, was purified by column chromatography over silica gel to obtain 5-[3-(acetyloxy)-2-oxo-1-piperidinyl]-N,N′-bis[2,3-bis(acetyloxy)propyl]-2,4,6-triiodo-1,3-benzenedicarboxamide as a white fluffy solid (10.3 g, 83% yield).

### Example 8d

### N,N′-Bis[2,3-dihydroxypropyl)-5-(3-hydroxy-2-oxo-1-piperidinyl)-2,4,6-triiodo-1,3-benzenedicarboxamide

A solution of 5-[3-(acetyloxy)-2-oxo-1-piperidinyl]-N,N′-bis[2,3-bis(acetyloxy)propyl]-2,4,6-triiodo-1,3-benzenedicarboxamide of example 8c (4 g, 4.3 mmol) in methanol (20 ml) was treated with a solution of sodium methoxide in methanol, prepared from sodium (30 mg, 1.3 mmol) and anhydrous methanol (20 ml), and the mixture was stirred at room temperature for 2 hours. The pH of the solution was adjusted down to 7 by the addition of Dowex-50 (H⁺) resin. The mixture was filtered and the filtrate was freed of the solvent to obtain the crude product. Purification by low pressure reverse phase column chromatography over the CHP-20P resin furnished analytically pure N,N′-Bis[2,3-dihydroxypropyl)-5-(3-hydroxy-2-oxo-1-piperidinyl)-2,4,6-triiodo-1,3-benzenedicarboxamide as a snow-white glassy solid (2.3 g, 78% yield).

### Example 9

### N,N′-Bis[2-hydroxy-1-(hydroxymethyl)ethyl]-5-(3-hydroxy-2-oxo-1-piperidinyl]-2,4,6-triiodo-1,3-benzenedicarboxamide

### Example 9a

### N,N′-Bis[2-(acetyloxy)-1-[(acetyloxy)methyl]ethyl]-5-[(2,5-dibromo-1-oxopentyl)amino]-2,4,6-triiodo-1,3-benzenedicarboxamide

2,5-Dibromopentanoyl bromide prepared as described in example 8a (15.9 g, 49.3 mmol) was added dropwise to a stirred solution of N,N′-bis[2-(acetyloxy)-1-[(acetyloxy)methyl]ethyl]-5-amino-2,4,6-triiodo-1,3-benzenedicarboxamide (33 g, 37.8 mmol) in dimethylacetamide (250 ml) at 0°. After the addition, the reaction mixture was stirred at 0° for 1 hour, and then at room temperature for 22 hours. The solution was then added slowly dropwise to a well stirred mixture of ice-water (2 L), when a white solid separated out. This was collected by filtration, washed with ice-water (3 x 50 ml), and dried in vacuo to obtain the crude product (40.1 g, 91.5% pure). Recrystallization from ethyl acetate furnished analytically pure N,N′-bis[2-(acetyloxy)-1-[(acetyloxy)methyl]ethyl]-5-[(2,5-dibromo-1-oxopentyl)amino]-2,4,6-triiodo-1,3-benzenedicarboxamide as an off-white crystalline compound (35.3 g, 84% yield), m.p. 240-243°.

### Example 9b

### N,N′-Bis[2-(acetyloxy)-1-[(acetyloxy)methyl]ethyl]-5-(3-bromo-2-oxo-1-piperidinyl)-2,4,6-triiodo-1,3-benzene-dicarboxamide

To a solution of N,N′-bis[2-(acetyloxy)-1-[(acetyloxy)methyl]ethyl]-5-[(2,5-dibromo-1-oxopentyl)amino]-2,4,6-triiodo-1,3-benzenedicarboxamide of example 9a (32.5 g, 29.1 mmol) in dimethylacetamide (250 ml), was added powdered potassium carbonate (25.2 g, 116.5 mmol). After stirring for 4 hours, the mixture was filtered. The filtrate was freed of the solvent in vacuo at 45°C and the resulting solid was redissolved in ethyl acetate (500 ml). The ethyl acetate solution was washed with water (2 x 50 ml) and saturated aqueous sodium chloride (1 x 100 ml), and dried over anhydrous magnesium sulfate. The solvent was removed in vacuo to obtain a white solid (29.5 g) which, upon crystallization from ethyl acetate (1.2 L), furnished analytically pure N,N′-bis[2-acetyloxy)-1-[(acetyloxy)-methyl]ethyl]-5-(3-bromo-2-oxo-1-piperidinyl)-2,4,6-triiodo-1,3-benzenedicarboxamide as a white crystalline solid (23.95 g, 79.5% yield), m.p. 231-3°.

### Example 9c

### N,N′-Bis[2-(acetyloxy)-1-[(acetyloxy)methyl]ethyl]-5-[3-(acetyloxy)-2-oxo-1-piperidinyl]-2,4,6-triiodo-1,3-benzene-dicarboxamide

A mixture of N,N′-bis[2-(acetyloxy)-1-[(acetyloxy)methyl]ethyl]-5-[3-bromo-2-oxo-1-piperidinyl]-2,4,6-triiodo-1,3-benzenedicarboxamide of example 9b (22.43 g, 21.67 mmol) and silver acetate (12.5 g, 74.8 mmol) in glacial acetic acid (400 ml) was refluxed for 28 hours. The reaction mixture was cooled to room temperature and then filtered. The filtrate was freed of the solvent in vacuo at 45° and the resulting residue was dissolved in ethyl acetate (500 ml). The ethyl acetate solution was washed successively with water (2 x 50 ml), saturated aqueous sodium bicarbonate (2 x 50 ml) and saturated aqueous sodium chloride (2 x 50 ml), and then dried over anhydrous magnesium sulfate. Removal of the solvent yielded a white powder (20.7 g), which upon crystallization from ethyl acetate (1.1 L), furnished analytically pure N,N′-bis [2-(acetyloxy)-1-[(acetyloxy)methyl]ethyl]-5-[3-(acetyloxy)-2-oxo-1-piperidinyl]-2,4,6-triiodo-1,3-benzenedicarboxamide as a white fluffy solid (15.45 g, 70% yield).

### Example 9d

### N,N′-Bis[2-hydroxy-1-(hydroxymethyl)ethyl]-5-(3-hydroxy-2-oxo-1-piperidinyl]-2,4,6-triiodo-1,3-benzenedicarboxamide

A solution of N,N′-bis[2-acetyloxy)-1-[(acetyloxy)methyl]ethyl]-5-(3-hydroxy-2-oxo-1-piperidinyl]-2,4,6-triiodo-1,3-benzenedicarboxamide of example 9c (2.0 g, 2.16 mmol) in methanol (10 ml) was treated with a solution of sodium methoxide in methanol, prepared from sodium (23 mg, 1 mmol) and anhydrous methanol (10 ml). The reaction mixture was stirred at room temperature for 3 hours. The pH of the solution was then adjusted to 7 by adding Dowex-50 (H⁺) resin. The resin was filtered off and the filtrate was freed of the solvent in vacuo to obtain the crude product as a colorless glass (1.47 g). Purification by low pressure reverse phase column chromatography over the CHP-20P resin afforded analytically pure N,N′-Bis[2-hydroxy-1-(hydroxymethyl)ethyl]-5-(3-hydroxy-2-oxo-1-piperidinyl]-2,4,6-triiodo-1,3-benzenedicarboxamide as a snow-white glassy solid (1.0 g, 68% yield).

### Example 10

### N,N′-Bis-[2,3-dihydroxy-1-propyl]-5-2-oxo-1-pyrrolidinyl]-2,4,6-triiodo-1,3-benzenedicarboxamide

### Example 10a

### N,N'-Bis-[2,3-bis-(acetyloxy)-1-propyl]-5-[4-chloro-1-oxobutyl]-2,4,6-triiodo-1,3-benzenedicarboxamide

To a solution of N,N′-bis-[2,3-bis-(acetyloxy)-1-propyl]-5-amino-2,4,6-triiodo-1,3-benzenedicarboxamide (87.3 grams, 100 mmol) in dry N,N-dimethylacetamide (370 ml) was added 4-chlorobutyl chloride (20.9 g, 148 mmol) via a syringe over a period of 2 minutes under N₂. The mixture was stirred for 68 hours at ambient temperature. The entire reaction mixture was poured into ice-water (800 ml) containing sodium bicarbonate(20g). The anilide precipitated as a tacky mass and the mixture was extracted with ethyl acetate (100 ml). The organic layer was removed and the aqueous layer was washed with ethyl acetate (2 x 300 ml). The organic layers were combined, washed with an equal volume of saturated aqueous sodium chloride solution in two batches and dried (magnesium sulfate). The solvents were removed and the resulting crude product was dried overnight at high vacuum to give an almost immobile orange syrup (114.5 g). Recrystallization of the crude product from ethylacetate/hexanes gave N,N′-bis-[2,3-bis-(acetyloxy)-1-propyl]-5-[4-chloro-1-oxobutyl]-2,4,6-triiodo-1,3-benzenedicarboxamide as an off-white powder (82.5 g, 84% yield), m.p. (211 - 214°).

### Example 10b

### N,N′-Bis-[2,3-bis-(acetyloxy)-1-propyl]-5-[2-oxo-1-pyrrolidinyl]-2,4,6-triiodo-1,3-benzenedicarboxamide

A solution of N,N′-bis-[2,3-bis-(acetyloxy)-1-propyl]-5-[4-chloro-1-oxobutyl]-2,4,6-triiodo-1,3-benzenedicarboxamide (55.36 g, 56.6 mmol) in N,N′-dimethylacetamide (500 ml) was cooled to -16.5° under N₂ and finely powdered potassium carbonate (54.76 g, 396.3 mmol) was added over a period of 2 minutes. The mixture was stirred for 44 hours at -16.5°. The heterogeneous reaction mixture that resulted was filtered under vacuum. The volatiles were evaporated from the filtrate under high vacuum at a bath temperature of 40 - 45°; near the end of the evaporation the bath temperature was raised to 50°C. The resulting thick, glassy syrup was dissolved in ethyl acetate (350 ml) and the solution was washed with an equal volume of distilled water. The layers were separated and the ethyl acetate layer was washed with water (350 ml) and then with saturated aqueous sodium chloride solution (350 ml). The organic layer was set aside and the first aqueous extract was back-extracted with ethyl acetate (300 ml). The resulting organic layer was washed with water (2 x 200 ml) and saturated aqueous sodium chloride solution (2 x 150 ml). The organic layers were combined and dried over magnesium sulfate and the solvents were removed. The crude product thus obtained was purified by flash chromatography over silica gel using ethyl acetate/dichloromethane 2/1, followed by ethyl acetate/dichloromethane 4/1, eluent. This gave N,N′-Bis-[2,3-bis-(acetyloxy)-1-propyl]-5-[2-oxo-1-pyrrolidinyl]-2,4,6-triiodo-1,3-benzenedicarboxamide (32.24 g, 60.5% yield) as an off-white foam. A sample, crystallized for analysis from ethyl acetate/hexanes, had m.p. 130 -133°.

### Example 10c

### N,N′-Bis-[2,3-dihydroxy-1-propyl]-5-[2-oxo-1-pyrrolidinyl]-2,4,6-triiodo-1,3-benzenedicarboxamide

N,N′-bis-[2,3-bis-(acetyloxy)-1-propyl]-5-[2-oxo-1-pyrrolidinyl]-2,4,6-triiodo-1,3-benzenedicarboxamide (24.88 g, 26.64 mmol) was dissolved in Mg°-dried methanol (200 ml) under N₂. To this solution was added a solution of sodium methoxide in methanol, prepared by dissolving sodium (0.158 g, 6.52 mmol) in Mg°-dried methanol (10 ml) at 0° under N₂ with stirring. The reaction mixture was stirred at ambient temperature for 7 hours.

BioRad Dowex AG-50 X8 resin (H+ form) (30 g) was added to the reaction mixture and the mixture was stirred for 20 minutes. The pH was adjusted to 4.4 by the addition of 4 drops of glacial acetic acid. The resin was removed by filtration and was rinsed with several 50 ml portions of methanol. The volatiles were removed and the residue was further dried under a vacuum of 0.5 mm of Hg overnight. The crude foam obtained was dissolved in distilled, deionized water and applied to a column of CHP-20 resin. The compound was eluted with 5.5% - 12% aqueous ethanol. The eluate was evaporated to give N,N′-bis-[2,3-dihydroxy-1-propyl]-5-[2-oxo-1-pyrrolidinyl]-2,4,6-triiodo-1,3-benzenedicarboxamide (13.87 g, 67.3% yield) as a white foam. A sample, crystallized for analysis from isopropanol, afforded the product as a white powder, (m.p. >265°).

### Example 11

### N,N′-Bis-[2-(hydroxy)-1-(hydroxymethyl)ethyl]-5-[2-(hydroxymethyl)]-5-oxo-1-pyrrolidinyl]-2,4,6-triiodo-1,3-benzenedicarboxamide

### Example 11a

### N,N′-Bis[2-(acetyloxy)-1-[(acetyloxy)methyl]ethyl]-2,4,6-triiodo-5-[(1-oxo-4-pentenoyl)amino]-1,3-benzenedicarboxamide

4-Pentenoyl chloride (11.9 g, 100 mmol) was added to a stirred solution of N,N′-bis[2-(acetyloxy)-1-[(acetyloxy)methyl]ethyl]-5-amino-2,4,6-triiodo-1,3-benzenedicarboxamide (47.75 g, 50 mmol) in dimethylacetamide (400 ml) at room temperature and the mixture was stirred for 16 hours. Dimethylacetamide was removed *in vacuo* and the residue dissolved in ethyl acetate (600 ml). The solution was washed with aqueous sodium bicarbonate (10%, 2 x 150 ml), water ( 2 x 100 ml), and brine (150 ml). The organic layer was dried and the solvent removed to obtain the crude product as an off-white solid. Purification by crystallization from a mixture of acetone (250 ml) and hexane (75 ml) afforded N,N′-bis[2-(acetyloxy)-1-[(acetyloxy)methyl]ethyl]-2,4,6-triiodo-5-[(1-oxo-4-pentenoyl)amino]-1,3-benzenedicarboxamide (42.5 g, yield, 81%).

### Example 11b

### N,N′-Bis[2-(acetyloxy)-1-[(acetyloxy)methyl]ethyl]-5-[2-(iodomethyl)-5-oxo-1-pyrrolidinyl]-2,4,6-triiodo-1,3-benzenedicarboxamide

To a solution of N,N′-bis[2-(acetyloxy)-1-[(acetyloxy)methyl]ethyl]-2,4,6-triiodo-5-[(1-oxo-4-pentenoyl)amino]-1,3-benzenedicarboxamide (19.1 g, 20 mmol) in methanol (200 ml) was added a solution of sodium methoxide in methanol, prepared by dissolving sodium (1.38 g, 60 mmol) in methanol (30 ml). The mixture was stirred for 30 minutes. The solvent was removed *in vacuo* and the residue was dissolved in a mixture of methanol and water (1:1, v/v; 200 ml). N-Iodosuccinimide (12.32 g, 60 mmol) was added and the mixture stirred at room temperature for 48 hours. The solvents were removed from the reaction mixture and the residue azeotroped with ethanol (3 x 150 ml). The residue was then dissolved in pyridine (150 ml) and treated with acetic anhydride ((20.4 g, 200 mmol) with stirring for 17 hours at room temperature. The excess of pyridine and acetic anhydride were removed *in vacuo*, the residue dissolved in ethyl acette (500 ml), and the resulting solution washed successively with water (200 ml), aqueous sodium thiosulfate (25%, 2 x 125 ml), and water (2 x 150 ml). The organic layer was dried and removal of the solvent afforded the product as a light yellow glassy solid (20.2 g). The crude product was purified by column chromatography over silica gel, using 25% hexane in ethyl acetate as the eluent, to obtain N,N′-bis[2-(acetyloxy)-1-[(acetyloxy)methyl]ethyl]-5-[2-(iodomethyl)-5-oxo-1-pyrrolidinyl]-2,4,6-triiodo-1,3-benzenedicarboxamide (13.2 g, 61.2 %) as a colorless powder.

### Example 11c

### N,N-Bis[2-(acetyloxy)-1-[(acetyloxy)methyl]ethyl]-5-[2-(acetyloxy)methyl)-5-oxo-1-pyrrolidinyl]-2,4,6-triiodo-1,3-benzenedicarboxamide

To a solution of N,N′-bis[2-(acetyloxy)-1-[(acetyloxy)methyl]ethyl]-5-[2-(iodomethyl)-5-oxo-1-pyrrolidinyl]-2,4,6-triiodo-1,3-benzenedicarboxamide (12.00 g, 110 mmol) in acetonitrile (150 ml) was added tetraethylammonium acetate (5.74 g, 22 mmol) and the mixture stirred at 50° for 18 hours. Acetonitrile was removed *in vacuo* at 60°, the residue dissolved in ethyl acetate (200 ml), and the resulting solution washed with brine (2 x 100 ml) and with water (100 ml). The ethyl acetate layer was dried and removal of the solvent afforded the crude product as a colorless glassy solid. This material was purified by column chromatography over silica gel, using 20% hexane in ethyl acetate as eluent, to obtain N,N-bis[2-(acetyloxy)-1-[(acetyloxy)methyl]ethyl]-5-[2-(acetyloxy)methyl)-5-oxo-1-pyrrolidinyl]-2,4,6-triiodo-1,3-benzenedicarboxamide as a colorless solid (9.4 g, 83.6% yield).

### Example 11d

### N,N′-Bis-[2-(hydroxy)-1-(hydroxymethyl)ethyl]-5-[2-(hydroxymethyl)]-5-oxo-1-pyrrolidinyl]-2,4,6-triiodo-1,3-benzenedicarboxamide

A solution of N,N-bis[2-(acetyloxy)-1-[(acetyloxy)methyl]ethyl]-5-[2-(acetyloxy)methyl)-5-oxo-1-pyrrolidinyl]-2,4,6-triiodo-1,3-benzenedicarboxamide (8.4 g, 8.3 mmol) in methanol (50 ml) was treated with a solution of sodium methoxide in methanol, prepared from sodium (190 mg, 8.3 mmol) and methanol (5 ml). The solution was stirred at room temperature for 1 hour. The pH of the solution was then adjusted to 7 with the ion exchange resin Dowex-50-(H)⁺. The resin was filtered off and the solvent removed to obtain the crude product as a glassy solid (6.45 g, 96.8%). This product was purified by reverse phase column chromatography using the nonionic CHP-20 resin and a solvent gradient varying from 100% deionized water to water containing 4% ethanol. The fractions containing the pure compound were combined and the solvents removed to obtain N,N′-Bis-[2-(hydroxy)-1-(hydroxymethyl)ethyl]-5-[2-(hydroxymethyl)]-5-oxo-1-pyrrolidinyl]-2,4,6-triiodo-1,3-benzenedicarboxamide as a colorless solid (5.88 g, yield 88%). M.P. 245 - 248°C.

### Example 12

### N,N′-Bis(2,3-Dihydroxypropyl)-5-[3-(hydroxymethyl)-5-oxo-4-morpholinyl]-2,4,6-triiodo-1,3-benzenedicarboxamide

### Example 12a

### N,N′-Bis[2,3-bis(acetyloxy)propyl]-2,4-6-triiodo-5-[((2-propenyloxy)acetyl)amino]-1,3-benzenedicarboxamide

Allyloxyacetyl chloride (4.0 gm, 30 mmol) was added dropwise to a stirred solution of N,N′-bis[2,3-bis(acetyloxy)propyl]-5-amino-2,4,6-triiodo-1,3-benzenedicarboxamide (22.69 g, 26 mmol) in dimethylacetamide (100 ml) at 0 - 5°. The reaction mixture was stirred at 5° for 30 minutes and at room temperature for 20 hours. It was then added dropwise to a well stirred mixture of ice-water (1L), when a gummy solid separated out. This was collected be decantation and dissolved in ethyl acetate (200 ml). The aqueous layer was extracted with ethyl acetate (2 x 200 ml). The combined organic layers were washed with water (2 x 100 ml), dried (MgSO₄), and concentrated to obtain a foamy solid (24.8 g). Purification by columm chromatography over silica gel using a gradient system of ethyl acetate/hexane (1:3 - 3:1) furnished N,N′-bis[2,3-bis(acetyloxy)propyl]-2,4-6-triiodo-5-[((2-propenyloxy)acetyl)amino]-1,3-benzenedicarboxamide as a white foamy solid 19.84 g, 77% yield).

### Example 12b

### N,N′-Bis[2,3-bis(acetyloxy)propyl]-2,4,6-triiodo-5-[3-iodomethyl)-5-oxo-4-morpholinyl]-1,3-benzenedicarboxamide

To a solution of N,N′-Bis[2,3-bis(acetyloxy)propyl]-2,4,6-triiodo-5-[3-iodomethyl)-5-oxo-4-morpholinyl]-1,3-benzenedicarboxamide (10.7 g, 11 mmol) in anhydrous methanol (100 ml), was added a solution of sodium methoxide in methanol, prepared by dissolving sodium metal (25 mg) in dry methanol (5 ml). After stirring for 4 hours, the solvent was removed and the residue was redissolved in a mixture of dioxane-methanol (150 ml, 1:3). An aqueous solution of sodium hydroxide (1M, 30 ml) was added, and the mixture stirred for 2 hours. N-Iodosuccinimide (3.38 g, 15 mmol) was then added in portions over a 2 hour period. After stirring further for 2 hours, more N-iodosuccinimide (2.25 g, 10 mmol) was added in portions to the clear yellow solution and the stirring continued for 40 hours. The pH of the solution was adjusted to 7 and the solvent removed *in vacuo* at 40°. The residue was stirred in a mixture of pyridine (25 ml, acetic anhydride (25 ml) and acetonitrile (20 ml) for 24 hours. The solvents were removed *in vacuo* and the brown residue, upon column chromatography over silica gel using a stepwise gradient of ethyl acetate-hexane (from 1:2 to 9:1), afforded pure N,N′-bis[2,3-bis(acetyloxy)propyl]-2,4,6-triiodo-5-[3-iodomethyl)-5-oxo-4-morpholinyl]-1,3-benzenedicarboxamide (7.5 g, 62% yield) as a white amorphous solid, m.p. 168 - 70°.

### Example 12c

### N,N′-Bis[2,3-bis(acetyloxy)propyl]-5-[3((Acetyloxy)-methyl)-5-oxo-4-morpholinyl]-2,4,6-triiodo-1,3-benzenedicarboxamide

To a solution of N,N′-bis[2,3-bis(acetyloxy)propyl]-5-[3((Acetyloxy)-methyl)-5-oxo-4-morpholinyl]-2,4,6-triiodo-1,3-benzenedicarboxamide 7.8 g, 7.1 mmol) in acetic acid (100 ml) was added silver acetate (4.0 g, 24 mmol). The mixture was stirred and refluxed for 24 hours. The mixture was then filtered to remove inorganic salts, which were washed with acetic acid (50 ml), followed by ethyl acetate (100 ml). The combined filtrate and washings were concentrated to dryness. The resulting residue was redissolved in ethyl acetate (200 ml). The ethyl acetate solution was washed successively with water (3 x 50 ml), saturated aqueous sodium bicarbonate (3 x 50 ml) and water (3 x 50 ml). Drying over anhydrous sodium sulfate, followed by removal of the solvent under reduced pressure, yielded the crude product as a light brown solid (7.2 g). Purification by column chromatography over silica gel using a gradient systems of ethyl acetate/hexane as eluent afforded N,N′-bis[2,3-bis(acetyloxy)propyl]-5-[3((Acetyloxy)-methyl)-5-oxo-4-morpholinyl]-2,4,6-triiodo-1,3-benzenedicarboxamide as an amorphous solid (5.36 g, 73% yield), m.p. 210 - 212°.

### Example 12d

### N,N′-Bis(2,3-dihydroxypropyl)-5-[3-(hydroxymethyl)-5-oxo-4-morpholinyl]-2,4,6-triiodo-1,3-benzenedicarboxamide

To a solution of N,N′-bis[2,3-bis(acetyloxy)propyl]-5-[3((Acetyloxy)-methyl)-5-oxo-4-morpholinyl]-2,4,6-triiodo-1,3-benzenedicarboxamide (5.15 g, 5 mmol) in anhydroous methanol (100 ml), was added a solution of sodium methoxide, prepared from sodium (25 mg) and anhydrous methanol (5 ml), and the mixture was stirred for 3 hours. The solution was adjusted to pH 7 by a slow addition of Dowex-50 (H⁺) resin and then filtered. The filtrate was concentrated *in vacuo* to obtain the crude product (3.85 g) as a white solid. The material was dissolved in water (40 ml) and loaded onto a column of CHP-20 diaion resin. The column was first eluted with water (1L) and then with a stepwise gradient of ethanol in water (1 - 8%). The fractions containing the pure product were combined and the solvents removed *in vacuo* to obtain N,N′-bis[2,3-bis(acetyloxy)propyl]-5-[3((Acetyloxy)-methyl)-5-oxo-4-morpholinyl]-2,4,6-triiodo-1,3-benzenedicarboxamide as a white microcrystalline solid (2.45 g, 60% yield, 99% purity). Recrystallization from ethanol/isopropanol (95/5) afforded the analytical sample as a white solid, m.p. 227 - 230°.

### Example 13

### N,N′-Bis(2,3-Dihydroxypropyl)-5-(2-oxo-1-piperidinyl)-2,4,6-triiodo-1,3-benzenedicarboxamide

### Example 13a

### N,N′-Bis[2,3-bis(Acetyloxy)propyl]-5-[(5-chloro-1-oxo-pentyl)amino]-2,4,6-triiodo-1,3-benzenedicarboxamide

5-Chloropentanyoyl chloride (5.1 g, 33 mmol) was added dropwise to a stirred solution of N,N′-bis[2,3-bis(acetyloxy)propyl]-5-amino-2,4,6-triiodo-1,3-benzenedicarboxamide (20.5 g, 23 mmol) in N,N′-dimethylacetamide (75 ml) at 0-5°. The reaction mixture was stirred at 5° for 1 hour, and then at room temperature for 30 hours. The solvent was removed *in vacuo* and the residue was dissolved in ethyl acetate (250 ml). The solution was washed with water (2 x 50 ml), followed by saturated aqueous NaCl (50 ml), and then dried over anhydrous sodium sulfate. Removal of the solvent *in vacuo* gave N,N′-bis[2,3-bis(acetyloxy)propyl]-5-[(5-chloro-1-oxopentyl)amino]-2,4,6-triiodo-1,3-benzenedicarboxamide as an off-white fluffy solid (22.1 g, yield 97%, purity 98.5%).

### Example 13b

### N,N′-bis[2,3-bis(acetyloxy)propyl]-5-(2-oxo-1-piperidinyl)-2,4,6-triiodo-1,3-benzenedicarboxamide

To a solution of N,N′-bis(acetyloxy)propyl]-5-[(5-chloro-1-oxopentyl)amino]-2,4,6-triiodo-1,3-benzenedicarboxamide (23,8 g, 24 mmol) in dimethylacetamide (200 ml) was added powdered anhydrous potassium carbonate (16.5 g, 120 mmol) and the mixture stirred for 4 hours at room temperature. The reaction mixture was filtered and the filtrate concentrated *in vacuo* to obtain a brown fluffy solid (23 g, purity 96.7 %, crude yield 99%). Purification of this material by column chromatography over silica gel afforded pure N,N′-bis[2,3-bis(acetyloxy)propyl]-5-(2-oxo-1-piperidinyl)-2,4,6-triiodo-1,3-benzenedicarboxamide as white fluffy solid (20.1 g, purity 99%, yield 87%, m.p. 130 - 134° (white needles from acetone/hexane).

### Example 13c

### N,N-bis(2,3-Dihydroxypropyl)-5-(2-oxo-1-piperidinyl)-2,4,6-triiodo-1,3-benzenedicarboxamide

To a solution of N,N′-bis[2,3-bis(acetyloxy)propyl]-5-(2-oxo-1-piperidinyl)-2,4,6-triiodo-1,3-benzenedicarboxamide (17.2 g, 18 mmol) in anhydrous methanol (100 ml) was added a solution of sodium methoxide, prepared by dissolving 48 mg of sodium in 2 ml of methanol. The mixture was stirred for 4 hours and the pH of the solution adjusted to 7 by the addition of AG 50W-X8 (H+ form). The resin was filtered off and the filtrate decolorized by treatment with activated charcoal and again filtered. The clear colorless filtrate, upon removal of the solvent, gave the crude product as a white solid (14.3 g, purity 99%), which was redissolved in water (100 ml) and purified by low pressure reverse phase column chromatography over the CHP-20 diaion resin. The fractions containing the pure product were combined and the solvents removed *in vacuo* to obtain N,N-bis(2,3-dihydroxypropyl)-5-(2-oxo-1-piperidinyl)-2,4,6-triiodo-1,3-benzenedicarboxamide as a white crystalline solid (11.95 g, yield 84.5%, purity 99.9%, m.p. 214 - 219°.

## Claims (Claims for the following Contracting State(s): AT, BE, CH, DE, DK, FR, GB, GR, IT, LI, LU, NL, SE)

1. A compound of the formula 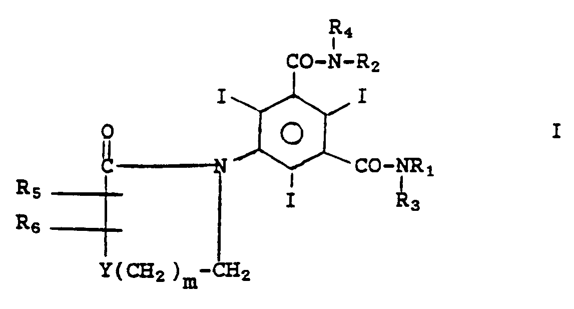 wherein Y is a single bond,-CH₂CH₂-, -CH₂O-, -OCH₂-, -N-CH₂-, -CH₂N-, 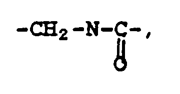 -CH₂-, -O- or -N-;
R₁ and R₂ are the same or different and are 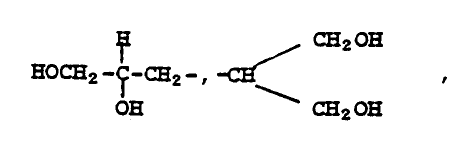 -CH₂CH₂OH 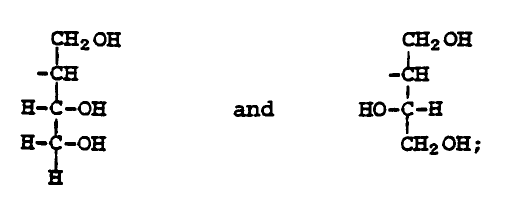 R₃ and R₄ are the same or different and are hydrogen, methyl or -CH₂CH₂OH; R₅ is hydrogen, alkyl, -CH₂CH₂OH, CH₂OH or OH and R₆ is alkyl, -CH₂CH₂OH, CH₂OH, OH or hydrogen and may be the same or different than R₅ and m is zero or one with the proviso that no methylene or methine carbon atom of the heterocyclic ring is attached to both a nitrogen and an oxygen atom, with the additional proviso that when Y is a single bond, m is not zero.

2. A compound according to claim 1 wherein Y is a single bond.

3. A compound according to claim 1 wherein Y is -CH₂CH₂-.

4. A compound according to claim 1 wherein Y is -CH₂O-.

5. A compound according to claim 1 wherein Y is -OCH₂-.

6. A compound according to claim 1 wherein Y is -N-CH₂-.

7. A compound according to claim 1 wherein Y is -CH₂-N-.

8. A compound according to claim 1 wherein Y

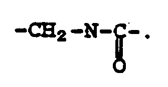

9. A compound according to claim 1 wherein Y is -CH₂-,

10. A compound according to claim 1 wherein Y is -O-.

11. A compound according to claim 1 wherein Y is -N-.

12. A compound according to claim 1, N,N'-Bis(2,3-dihydroxypropyl)-5-[4-(hydroxyethyl)-2-oxo-3-oxazolidinyl]-2,4,6-triiodo-1,3-benzenedicarboxamide.

13. A compound according to claim 1, N,N'-bis-(2,3-dihydroxypropyl)-5-[4-(R)-(hydroxymethyl)-2-oxo-3-oxazolidinyl]-2,4,6-triiodo-1,3-benzenedicarboxamide.

14. A compound according to claim 1, N,N'-Bis[2-hydroxy-1-(hydroxymethyl)ethyl]-2,4,6-triiodo-5-[4-(hydroxymethyl)-2-oxo-3-oxazolidinyl]-1,3-benzenedicarboxamide.

15. A compound according to claim 1, N,N'-Bis-[2-hydroxy-1-(hydroxymethyl)ethyl]-5-[3-hydroxy-2-oxo-1-pyrrolidinyl]-2,4,6-triiodo-1,3-benzenedicarboxamide.

16. A compound according to claim 1, N,N'-Bis-[2,3-dihydroxy-propyl]-5-[3-hydroxy-2-oxo-1-pyrrolidiny]-2,4,6-triiodo-1,3-benzenedicarboxamide.

17. A compound according to claim 1, 5-[2-(Hydroxymethyl)-5-oxo-1-pyrrolidinyl]-N,N'-bis[2,3-dihydroxypropyl]-2,4,6-triiodo-1,3-benzenedicarboxamide.

18. A compound according to claim 1, N,N'-Bis[2-Hydroxy-1-(hydroxymethyl)ethyl]-5-[3-(hydroxymethyl)-5-oxo-4-morpholinyl]-2,4,6-triiodo-1,3-benzenedicarboxamide.

19. A compound according to claim 1, N,N'-Bis[2,3-dihydroxypropyl)-5-(3-hydroxy-2-oxo-1-piperidinyl)-2,4,6-triiodo-1,3-benzenedicarboxamide.

20. A compound according to claim 1, N,N′-Bis[2-hydroxy-1-(hydroxymethyl)ethyl]-5-(3-hydroxy-2-oxo-1-piperidinyl]-2,4,6-triiodo-1,3-benzenedicarboxamide.

21. A compound according to claim 1, N,N′-Bis-[2,3-dihydroxy-1-propyl]-5-2-oxo-1-pyrrolidinyl]-2,4,6-triiodo-1,3-benzenedicarboxamide.

22. A compound according to claim 1, N,N′-Bis-[2-(hydroxy)-1-(hydroxymethyl)ethyl]-5-[2-(hydroxymethyl)]-5-oxo-1-pyrrolidinyl]-2,4,6-triiodo-1,3-benzenedicarboxamide.

23. A compound according to claim 1, N,N′-Bis(2,3-Dihydroxypropyl)-5-[3-(hydroxymethyl)-5-oxo-4-morpholinyl]-2,4,6-triiodo-1,3-benzenedicarboxamide.

24. A compound according to claim 1, N,N′-Bis(2,3-Dihydroxypropyl)-5-(2-oxo-1-piperidinyl)-2,4,6-triiodo-1,3-benzenedicarboxamide.

25. A method of using the compound according to any preceding claim as a contrast agent for determining conditions within a body cavity.

26. A method according to claim 25, wherein the contrast agent is present in a physiologically acceptable medium at a concentration in the range of about 50 to 500 mg of Iodine/ml.

27. A method of preparing lactams by reacting 1-oxo-ω-alkenylamino or 1-oxo-ω-oxaalkenylamino derivatives of 2,4,6-triiodo 1,3-benzenedicarboxamides with iodinating agents in the presence of a base.

28. A method according to claim 27 wherein the iodinating agent is N-iodosuccinimide.

29. A method according to claim 27 or 28 wherein the base is sodium hydroxide, sodium methoxide or sodium hydride.

30. A method of preparing a compound according to any one of claims 1-24, which comprises a ring closure reaction of the optionally substituted methylene group onto the nitrogen atom in the heterocyclic ring structure of formula I in claim 1, the carbon atom of the methylene group being activated for ring closure, preferably by an epoxy group, a halogen atom, or an olefinic bond.

31. A compound according to any one of claims 1-24 for use as a contrast agent for diagnostic purposes.

32. Use of a compound according to any one of claims 1-24 for the manufacture of a contrast agent formulation for diagnostic purposes.

## Claims (Claims for the following Contracting State(s): ES)

1. A method of preparing a compound of the formula 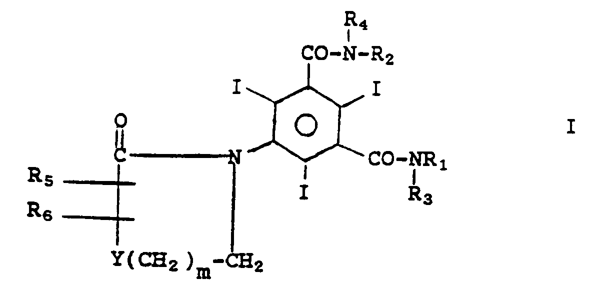 wherein Y is a single bond,-CH₂CH₂-, -CH₂O-, -OCH₂-, -N-CH₂-, -CH₂N-, 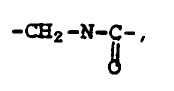 -CH₂-, -O- or -N-;
R₁ and R₂ are the same or different and are 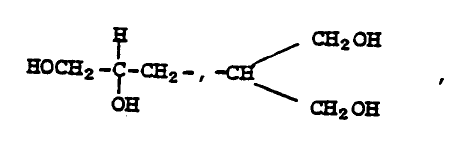 -CH₂CH₂OH 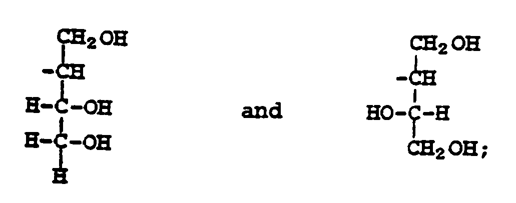 R₃ and R₄ are the same or different and are hydrogen, methyl or -CH₂CH₂OH; R₅ is hydrogen, alkyl, -CH₂CH₂OH, CH₂OH or OH and R₆ is alkyl, -CH₂CH₂OH, CH₂OH, OH or hydrogen and may be the same or different than R₅ and m is zero or one with the proviso that no methylene or methine carbon atom of the heterocyclic ring is attached to both a nitrogen and an oxygen atom, with the additional proviso that when Y is a single bond, m is not zero, which comprises a ring closure reaction of the optionally substituted methylene group onto the nitrogen atom in the heterocyclic ring structure, the carbon atom of the methylene group being activated for ring closure, preferably by an epoxy group, a halogen atom, or an olefinic bond.

2. A method according to claim 1 wherein Y is a single bond.

3. A method according to claim 1 wherein Y is -CH₂CH₂-.

4. A method according to claim 1 wherein Y is -CH₂O-.

5. A method according to claim 1 wherein Y is -OCH₂-.

6. A method according to claim 1 wherein Y is -N-CH₂-.

7. A method according to claim 1 wherein Y is -CH₂-N-.

8. A method according to claim 1 wherein Y

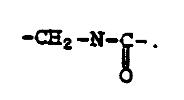

9. A method according to claim 1 wherein Y is -CH₂-,

10. A method according to claim 1 wherein Y is -O-.

11. A method according to claim 1 wherein Y is -N-.

12. A method according to claim 1 wherein the product is N,N'-Bis(2,3-dihydroxypropyl)-5-[4-(hydroxymethyl)-2-oxo-3-oxazolindyl]-2,4,6-triiodo-1,3-benzenedicarboxamide.

13. A method according to claim 1 wherein the product is N,N'-Bis-(2,3-dihydroxypropyl)-5-[4-(R)-(hydroxymethyl)-2-oxo-3-oxazolidinyl]-2,4,6-triiodo-1,3-benzenedicarboxamide.

14. A method according to claim 1 wherein the product is N,N'-Bis[2-hydroxy-1-(hydroxymethyl)ethyl]-2,4,6-triiodo-5-[4-(hydroxymethyl)-2-oxo-3-oxazolidinyl]-1,3-benzenedicarboxamide.

15. A method according to claim 1 wherein the product is N,N'-Bis-[2-hydroxy-1-(hydroxymethyl)ethyl]-5-[3-hydroxy-2-oxo-1-pyrrolidinyl]-2,4,6-triiodo-1,3-benzenedicarboxamide.

16. A method according to claim 1 wherein the product is N,N'-Bis-[2,3-dihydroxy-propyl]-5-[3-hydroxy-2-oxo-1-pyrrolidinyl]-2,4,6-triiodo-1,3-benzenedicarboxamide.

17. A method according to claim 1 wherein the product is 5-[2-(Hydroxymethyl)-5-oxo-1-pyrrolidinyl]-N,N'-bis[2,3-dihydroxypropyl]-2,4,6-triiodo-1,3-benzenedicarboxamide.

18. A method according to claim 1 wherein the product is N,N'-Bis[2-Hydroxy-1-(hydroxymethyl)ethyl]-5-[3-(hydroxymethyl)-5-oxo-4-morpholinyl]-2,4,6-triiodo-1,3-benzenedicarboxamide.

19. A method according to claim 1 wherein the product is N,N'-Bis[2,3-dihydroxypropyl)-5-(3-hydroxy-2-oxo-1-piperidinyl)-2,4,6-triiodo-1,3-benzenedicarboxamide.

20. A method according to claim 1 wherein the product is N,N'-Bis[2-hydroxy-1-(hydroxymethyl)ethyl]-5-(3-hydroxy-2-oxo-1-piperidinyl]-2,4,6-triiodo-1,3-benzenedicarboxamide.

21. A method according to claim 1 wherein the product is N,N'-Bis-[2,3-dihydroxy-1-propyl]-5-2-oxo-1-pyrrolidinyl]-2,4,6-triiodo-1,3-benzenedicarboxamide.

22. A method according to claim 1 wherein the product is N,N'-Bis-[2-(hydroxy)-1-(hydroxymethyl)ethyl]-5-[2-(hydroxymethyl)]-5-oxo-1-pyrrolidinyl]-2,4,6-triiodo-1,3-benzenedicarboxamide.

23. A method according to claim 1 wherein the product is N,N'-Bis(2,3-Dihydroxypropyl)-5-[3-(hydroxymethyl)-5-oxo-4-morpholinyl]-2,4,6-triiodo-1,3-benzenedicarboxamide.

24. A method according to claim 1 wherein the product is N,N'-Bis(2,3-Dihydroxypropyl)-5-(2-oxo-1-piperidinyl)-2,4,6-triiodo-1,3-benzenedicarboxamide.

25. A method of using the compound according to any preceding claim as a contrast agent for determining conditions within a body cavity.

26. A method according to claim 25, wherein the contrast agent is present in a physiologically acceptable medium at a concentration in the range of about 50 to 500 mg of Iodine/ml.

27. A method of preparing lactams by reacting 1-oxo-ω-alkenylamino or 1-oxo-ω-oxaalkenylamino derivatives of 2,4,6-triiodo 1,3-benzenedicarboxamides with iodinating agents in the presence of a base.

28. A method according to claim 27 wherein the iodinating agent is N-iodosuccinimide.

29. A method according to claim 27 or 28 wherein the base is sodium hydroxide, sodium methoxide or sodium hydride.

## Patentansprüche (Patentansprüche für folgende(n) Vertragsstaat(en): AT, BE, CH, DE, DK, FR, GB, GR, IT, LI, LU, NL, SE)

1. Verbindung mit der Formel 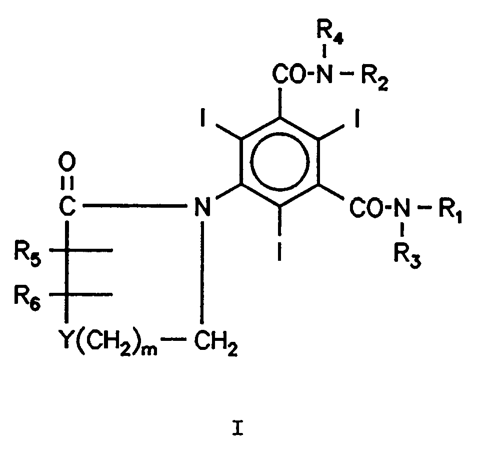 , bei der Y eine Einfachbindung, -CH₂CH₂-, -CH₂O-, -OCH₂-, -N-CH₂-, -CH₂N-, -CH₂-N-C(O)-, -CH₂-, -O- oder -N- ist,
R₁ und R₂ gleich oder verschieden und 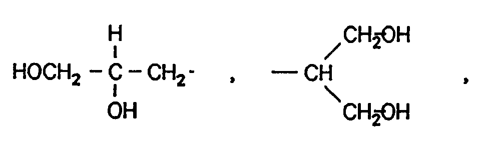 ―CH₂-CH₂-OH , 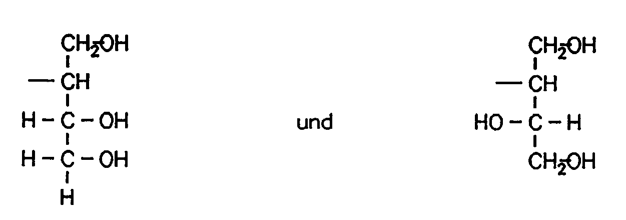 sind, R₃ und R₄ gleich oder verschieden und Wasserstoff, Methyl oder -CH₂CH₂OH sind, R₅ Wasserstoff, Alkyl, -CH₂CH₂OH, -CH₂OH oder -OH ist und R₆ Alkyl, -CH₂CH₂OH, -CH₂OH, -OH oder Wasserstoff ist und gleich oder von R₅ verschieden sein kann und m 0 oder 1 ist, mit der Maßgabe, daß kein Methylen- oder Methin-Kohlenstoffatom des heterocyclischen Ringes sowohl an ein Stickstoff- als auch an ein Sauerstoffatom gebunden ist, mit der zusätzlichen Maßgabe, daß, wenn Y eine Einfachbindung ist, m nicht 0 ist.

2. Verbindung nach Anspruch 1, bei der Y eine Einfachbindung ist.

3. Verbindung nach Anspruch 1, bei der Y -CH₂CH₂- ist.

4. Verbindung nach Anspruch 1, bei der Y -CH₂O- ist.

5. Verbindung nach Anspruch 1, bei der Y -OCH₂- ist.

6. Verbindung nach Anspruch 1, bei der Y -N-CH₂- ist.

7. Verbindung nach Anspruch 1, bei der Y -CH₂-N- ist.

8. Verbindung nach Anspruch 1, bei der Y -CH₂-N-C(O)- ist.

9. Verbindung nach Anspruch 1, bei der Y -CH₂- ist.

10. Verbindung nach Anspruch 1, bei der Y -O- ist.

11. Verbindung nach Anspruch 1, bei der Y -N- ist.

12. Verbindung nach Anspruch 1, N,N'-Bis(2,3-dihydroxypropyl)-5-[4-(hydroxymethyl)-2-oxo-3-oxazolidinyl]-2,4,6-triiodo-1,3-benzoldicarbonsäureamid.

13. Verbindung nach Anspruch 1, N,N'-Bis(2,3-dihydroxypropyl)-5-[4-(R)-(hydroxymethyl)-2-oxo-3-oxazolidinyl]-2,4,6-triiodo-1,3-benzoldicarbonsäureamid.

14. Verbindung nach Anspruch 1, N,N'-Bis[2-hydroxy-1-(hydroxymethyl)ethyl]-2,4,6-triiodo-5-[4-(hydroxymethyl)-2-oxo-3-oxazolidinyl]-1,3-benzoldicarbonsäureamid.

15. Verbindung nach Anspruch 1, N,N'-Bis-[2-hydroxy-1-(hydroxymethyl)ethyl]-5-[3-hydroxy-2-oxo-1-pyrrolidinyl]-2,4,6-triiodo-1,3-benzoldicarbonsäureamid.

16. Verbindung nach Anspruch 1, N,N'-Bis-[2,3-dihydroxypropyl]-5-[3-hydroxy-2-oxo-1-pyrrolidinyl]-2,4,6-triiodo-1,3-benzoldicarbonsäureamid.

17. Verbindung nach Anspruch 1, 5-[2-(Hydroxymethyl)-5-oxo-1-pyrrolidinyl]-N,N'-bis[2,3-dihydroxypropyl]-2,4,6-triiodo-1,3-benzoldicarbonsäureamid.

18. Verbindung nach Anspruch 1, N,N'-Bis[2-hydroxy-1-(hydroxymethyl)ethyl]-5-[3-(hydroxymethyl)-5-oxo-4-morpholinyl]-2,4,6-triiodo-1,3-benzoldicarbonsäureamid.

19. Verbindung nach Anspruch 1, N,N'-Bis[2-dihydroxypropyl]-5-(3-hydroxy-2-oxo-1-piperidinyl)-2,4,6-triiodo-1,3-benzoldicarbonsäureamid.

20. Verbindung nach Anspruch 1, N,N'-Bis[2-hydroxy-1-(hydroxymethyl)ethyl]-5-[3-hydroxy-2-oxo-1-piperidinyl]-2,4,6-triiodo-1,3-benzoldicarbonsäureamid.

21. Verbindung nach Anspruch 1, N,N'-Bis-[2,3-dihydroxy-1-propyl]-5-[2-oxo-1-pyrrolidinyl]-2,4,6-triiodo-1,3-benzoldicarbonsäureamid.

22. Verbindung nach Anspruch 1, N,N'-Bis-[2-(hydroxy)-1-(hydroxymethyl)ethyl]-5-[2-(hydroxymethyl)-5-oxo-1-pyrrolidinyl]-2,4,6-triiodo-1,3-benzoldicarbonsäureamid.

23. Verbindung nach Anspruch 1, N,N'-Bis(2,3-dihydroxypropyl)-5-[3-(hydroxymethyl)-5-oxo-4-morpholinyl]-2,4,6-triiodo-1,3-benzoldicarbonsäureamid.

24. Verbindung nach Anspruch 1, N,N'-Bis(2,3-dihydroxypropyl)-5-(2-oxo-1-piperidinyl)-2,4,6-triiodo-1,3-benzoldicarbonsäureamid.

25. Verfahren zur Verwendung der Verbindung gemäß einem der vorhergehenden Ansprüche als Kontrastmittel zur Bestimmung der Verhältnisse innerhalb einer Körperhöhle.

26. Verfahren nach Anspruch 25, bei dem das Kontrastmittel in einem physiologisch verträglichen Medium bei einer Konzentration im Bereich von etwa 50 bis 500 mg Jod/ml vorhanden ist.

27. Verfahren zur Herstellung von Lactamen durch Reaktion von 1-Oxo-ω-alkenylamino- oder 1-Oxo-ω-oxaalkenylamino-Derivaten von 2,4,6-Triiodo-1,3-benzoldicarbonsäureamiden mit Jodierungsmitteln in Gegenwart einer Base.

28. Verfahren nach Anspruch 27, bei dem das Jodierungsmittel N-Jodsuccinimid ist.

29. Verfahren nach Anspruch 27 oder 28, bei dem die Base Natriumhydroxid, Natriummethoxid oder Natriumhydrid ist.

30. Verfahren zur Darstellung einer Verbindung nach einem der Ansprüche 1 bis 24, das eine Ringschlußreaktion der gegebenenfalls substituierten Methylengruppe und dem Stickstoffatom in der heterocyclischen Ringstruktur von Formel I in Anspruch 1 umfaßt, wobei das Kohlenstoffatom der Methylengruppe zum Ringschluß aktiviert ist, vorzugsweise durch eine Epoxidgruppe, ein Halogenatom oder eine olefinische Bindung.

31. Verbindung nach einem der Ansprüche 1 bis 24 zur Verwendung als Kontrastmittel für diagnostische Zwecke.

32. Verwendung einer Verbindung gemäß einem der Ansprüche 1 bis 24 zur Herstellung einer Kontrastmittel-Formulierung für diagnostische Zwecke.

## Patentansprüche (Patentansprüche für folgende(n) Vertragsstaat(en): ES)

1. Verfahren zur Herstellung einer Verbindung der Formel 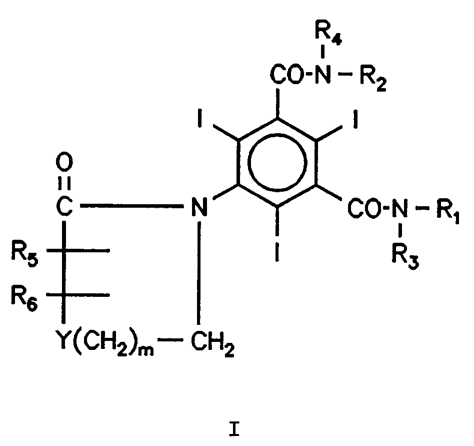 , bei der Y eine Einfachbindung, -CH₂CH₂-, -CH₂O-, -OCH₂-, -N-CH₂-, -CH₂N-, -CH₂-N-C(O)-, -CH₂-, -O- oder -N- ist,
R₁ und R₂ gleich oder verschieden und 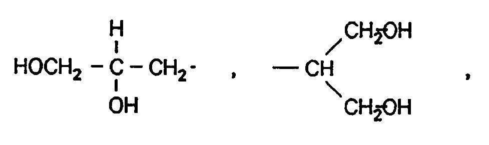 ―CH₂-CH₂-OH , 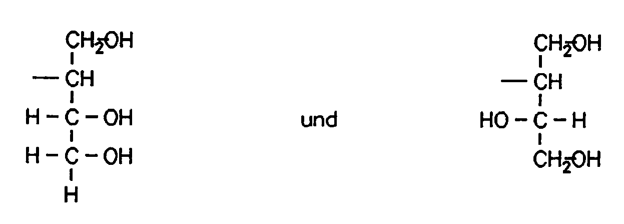 sind, R₃ und R₄ gleich oder verschieden und Wasserstoff, Methyl oder -CH₂CH₂OH sind, R₅ Wasserstoff, Alkyl, -CH₂CH₂OH, -CH₂OH oder -OH ist und R₆ Alkyl, -CH₂CH₂OH, -CH₂OH, -OH oder Wasserstoff ist und gleich oder von R₅ verschieden sein kann und m 0 oder 1 ist, mit der Maßgabe, daß kein Methylen- oder Methin-Kohlenstoffatom des heterocyclischen Ringes sowohl an ein Stickstoff- als auch an ein Sauerstoffatom gebunden ist, mit der zusätzlichen Maßgabe, daß, wenn Y eine Einfachbindung ist, m nicht 0 ist, das eine Ringschlußreaktion der gegebenenfalls substituierten Methylengruppe und dem Stickstoffatom in der heterocyclischen Ringstruktur umfaßt, wobei das Kohlenstoffatom der Methylengruppe zum Ringschluß aktiviert ist, vorzugsweise durch eine Epoxidgruppe, ein Halogenatom oder eine olefinische Bindung.

2. Verfahren nach Anspruch 1, bei dem Y eine Einfachbindung ist.

3. Verfahren nach Anspruch 1, bei dem Y -CH₂CH₂- ist.

4. Verfahren nach Anspruch 1, bei dem Y -CH₂O- ist.

5. Verfahren nach Anspruch 1, bei dem Y -OCH₂- ist.

6. Verfahren nach Anspruch 1, bei dem Y -N-CH₂- ist.

7. Verfahren nach Anspruch 1, bei dem Y -CH₂-N- ist.

8. Verfahren nach Anspruch 1, bei dem Y -CH₂-N-C(O)- ist.

9. Verfahren nach Anspruch 1, bei dem Y -CH₂- ist.

10. Verfahren nach Anspruch 1, bei dem Y -O- ist.

11. Verfahren nach Anspruch 1, bei dem Y -N- ist.

12. Verfahren nach Anspruch 1, bei dem das Produkt N,N'-Bis(2,3-dihydroxypropyl)-5-[4-(hydroxymethyl)-2-oxo-3-oxazolidinyl]-2,4,6-triiodo-1,3-benzoldicarbonsäureamid ist.

13. Verfahren nach Anspruch 1, bei dem das Produkt N,N'-Bis-(2,3-dihydroxypropyl)-5-[4-(R)-(hydroxymethyl)-2-oxo-3-oxazolidinyl]-2,4,6-triiodo-1,3-benzoldicarbonsäureamid ist.

14. Verfahren nach Anspruch 1, bei dem das Produkt N,N'-Bis[2-hydroxy-1-(hydroxymethyl)ethyl]-2,4,6-triiodo-5-[4-(hydroxymethyl)-2-oxo-3-oxazolidinyl]-1,3-benzoldicarbonsäureamid ist.

15. Verfahren nach Anspruch 1, bei dem das Produkt N,N'-Bis-[2-hydroxy-1-(hydroxymethyl)ethyl]-5-[3-hydroxy-2-oxo-1-pyrrolidinyl]-2,4,6-triiodo-1,3-benzoldicarbonsäureamid ist.

16. Verfahren nach Anspruch 1, bei dem das Produkt N,N'-Bis-[2,3-dihydroxypropyl]-5-[3-hydroxy-2-oxo-1-pyrrolidinyl]-2,4,6-triiodo-1,3-benzoldicarbonsäureamid ist.

17. Verfahren nach Anspruch 1, bei dem das Produkt 5-[2-(Hydroxymethyl)-5-oxo-1-pyrrolidinyl]-N,N'-bis[2,3-dihydroxypropyl]-2,4,6-triiodo-1,3-benzoldicarbonsäureamid ist.

18. Verfahren nach Anspruch 1, bei dem das Produkt N,N'-Bis[2-hydroxy-1-(hydroxymethyl)ethyl]-5-[3-(hydroxymethyl)-5-oxo-4-morpholinyl]-2,4,6-triiodo-1,3-benzoldicarbonsäureamid ist.

19. Verfahren nach Anspruch 1, bei dem das Produkt N,N'-Bis[2,3-dihydroxypropyl]-5-(3-hydroxy-2-oxo-1-piperidinyl)-2,4,6-triiodo-1,3-benzoldicarbonsäureamid ist.

20. Verfahren nach Anspruch 1, bei dem das Produkt N,N'-Bis[2-hydroxy-1-(hydroxymethyl)ethyl]-5-[3-hydroxy-2-oxo-1-piperidinyl]-2,4,6-triiodo-1,3-benzoldicarbonsäureamid ist.

21. Verfahren nach Anspruch 1, bei dem das Produkt N,N'-Bis-[2,3-dihydroxy-1-propyl]-5-[2-oxo-1-pyrrolidinyl]-2,4,6-triiodo-1,3-benzoldicarbonsäureamid ist.

22. Verfahren nach Anspruch 1, bei dem das Produkt N,N'-Bis-[2-(hydroxy)-1-(hydroxymethyl)ethyl]-5-[2-(hydroxymethyl)-5-oxo-1-pyrrolidinyl]-2,4,6-triiodo-1,3-benzoldicarbonsäureamid ist.

23. Verfahren nach Anspruch 1, bei dem das Produkt N,N'-Bis(2,3-dihydroxypropyl)-5-[3-(hydroxymethyl)-5-oxo-4-morpholinyl]-2,4,6-triiodo-1,3-benzoldicarbonsäureamid ist.

24. Verfahren nach Anspruch 1, bei dem das Produkt N,N'-Bis(2,3-dihydroxypropyl)-5-(2-oxo-1-piperidinyl)-2,4,6-triiodo-1,3-benzoldicarbonsäureamid.

25. Verfahren zur Verwendung der Verbindung gemäß einem der vorhergehenden Ansprüche als Kontrastmittel zur Bestimmung der Verhältnisse innerhalb einer Körperhöhle.

26. Verfahren nach Anspruch 25, bei dem das Kontrastmittel in einem physiologisch verträglichen Medium bei einer Konzentration im Bereich von etwa 50 bis 500 mg Jod/ml vorhanden ist.

27. Verfahren zur Herstellung von Lactamen durch Reaktion von 1-Oxo-ω-alkenylamino- oder 1-Oxo-ω-oxaalkenylamino-Derivaten von 2,4,6-Triiodo-1,3-benzoldicarbonsäureamiden mit Jodierungsmitteln in Gegenwart einer Base umgesetzt werden.

28. Verfahren nach Anspruch 27, bei dem das Jodierungsmittel N-Jodsuccinimid ist.

29. Verfahren nach Anspruch 27 oder 28, bei dem die Base Natriumhydroxid, Natriummethoxid oder Natriumhydrid ist.

## Revendications (Revendications pour l'(les) Etat(s) contractant(s) suivant(s): AT, BE, CH, DE, DK, FR, GB, GR, IT, LI, LU, NL, SE)

1. Composés de formule 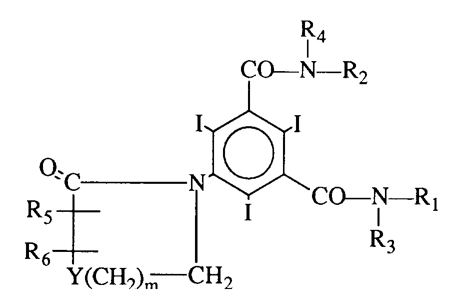 dans laquelle Y est une liaison simple, -CH₂-CH₂-, -CH₂O-, -OCH₂-, -N-CH₂-, -CH₂N-, -CH₂-H-CO-, -CH₂-, -O-, ou -N-;
R1 et R2, identiques ou différents, ont les formules: 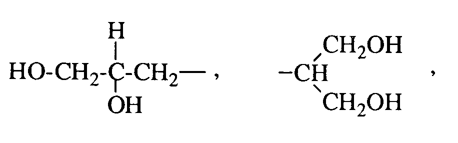 -CH₂CH₂OH 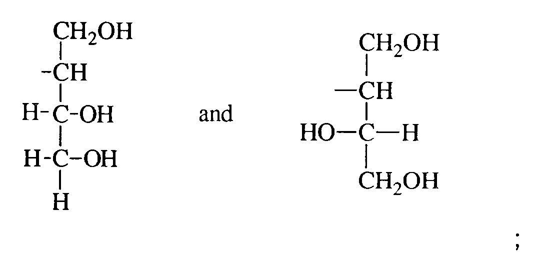 R3 et R4, identiques ou différents sont des restes hydrogène, méthyle, ou -CH₂CH₂OH; R5 et R6, identiques ou différents, sont des restes hydrogène, alcoyle, -CH₂CH₂OH, CH₂OH, ou OH, et m est 0 ou 1, étant donné qu'aucun atome de carbone méthénique ou méthinique de l'hétérocycle n'est simultanément relié à des atomes d'oxygène et d'azote, et étant encore donné que lorsque Y est une simple liaison, m n'est pas zero.

2. Composé suivant la revendication 1, où Y est une liaison simple.

3. Composé suivant la revendication 1, où Y est -CH2CH2-.

4. Composé suivant la revendication 1, où Y est -CH2O-.

5. Composé suivant la revendication 1, où Y est -OCH2-.

6. Composé suivant la revendication 1, où Y est -NCH2-.

7. Composé suivant la revendication 1, où Y est -CH2N-.

8. Composé suivant la revendication 1, où Y est -CH2-NCO-.

9. Composé suivant la revendication 1, où Y est -CH2-.

10. Composé suivant la revendication 1, où Y est -O-.

11. Composé suivant la revendication 1, où Y est -N-.

12. Composé N,N'-bis(2,3-dihydroxypropyl)-5[4-(hydroxyméthyl)-2-oxo-3-oxazolodinyl]-2,4,6-triiodo-1,3-benzènedicarboxamide suivant la revendication 1.

13. Composé N,N'-bis(2,3-dihydroxypropyl)-5[4-(R)-(hydroxyéthyl)-2-oxo-3-oxazolinidyl]-2,4,6-triiodo-1,3-benzènedicarboxamide suivant la revendication 1.

14. Composé N,N'-bis[2-hydroxy-1-(hydroxyéthyl)éthyl]-2,4,6-triiodo-5-[4-(hydroxyméthyl)-2-oxo-3-oxazolinidyl]-1,3-benzènedicarboxamide suivant la revendication 1.

15. Composé N,N'-bis[2-hydroxy-1-(hydroxyméthyl)éthyl]-5-[3-hydroxy-2-oxo-1-pyrrolidinyl]-2,4,6-triiodo-1,3-benzènedicarboxamide suivant la revendication 1.

16. Composé N,N'-bis(2,3-dihydroxypropyl)-5-[3-hydroxy-2-oxo-1-pyrrolidinyl]-2,4,6-triiodo-1,3-benzènedicarboxamide suivant la revendication 1.

17. Composé N,N'-bis(2,3-dihydroxypropyl)-5[2-(hydroxyméthyl)-5-oxo-1-pyrrolidinyl]-2,4,6-triiodo-1,3-benzènedicarboxamide suivant la revendication 1.

18. Composé N,N'-bis[2-hydroxy-1-(hydroxyméthyl)éthyl]-5-[3-hydroxyméthyl-5-oxo-4-morpholinyl]-2,4,6-triiodo-1,3-benzènedicarboxamide suivant la revendication 1.

19. Composé N,N'-bis[2,3-dihydroxypropyl]-5-(3-hydroxy-2-oxo-1-piperidinyl)-2,4,6-triiodo-1,3-benzènedicarboxamide suivant la revendication 1.

20. Composé N,N'-bis[2-hydroxy-1(hydroxyméthyl)éthylpropyl]-5-(3-hydroxy-2-oxo-1-piperidinyl)-2,4,6-triiodo-1,3-benzènedicarboxamide suivant la revendication 1.

21. Composé N,N'-bis[2,3-dihydroxy-1-propyl]-5-(3-hydroxy-2-oxo-1-pyrrolidinyl)-2,4,6-triiodo-1,3-benzènedicarboxamide suivant la revendication 1.

22. Composé N,N'-bis[2-hydroxy-1(hydroxyméthyl)éthyl]-5-(2-hydroxyméthyl-5-oxo-1-pyrrolidinyl)-2,4,6-triiodo-1,3-benzènedicarboxamide suivant la revendication 1.

23. Composé N,N'-bis[2,3-dihydroxypropyl]-5-[3-(hydroxyméthyl)-5-oxo-4-morpholinyl]-2,4,6-triiodo-1,3-benzènedicarboxamide suivant la revendication 1.

24. Composé N,N'-bis[2,3-dihydroxypropyl]-5-(2-oxo-1-piperidinyl)-2,4,6-triiodo-1,3-benzènedicarboxamide suivant la revendication 1.

25. Utilisation des composés suivant une quelconque des revendications précédentes comme agent de contraste pour déterminer les conditions régnant à l'intérieur d'une cavité du corps.

26. Utilisation suivant la revendication 25, selon laquelle l'agent de contraste se trouve dans un milieu physiologiquement acceptable à une concentration de l'ordre d'environ 50 à 500 mg d'iode/ml.

27. Procédé de préparation de lactames par la réaction de dérivés 1-oxo-ω-alcoylénylamino-, ou 1-oxo-ω-oxaalcoylénamino- de 2,4,6-triodo-1,3-benzènedicarboxamides avec des agents iodants en présence d'une base.

28. Procédé suivant la revendication 27, dans lequel l'agent iodant est la N-iodosuccinimide.

29. Procédé suivant les revendications 27 ou 28, dans lequelles la base est l'hydroxyde de sodium, le méthylate de sodium ou l'hydrure de sodium.

30. Procédé de préparation d'un composé selon une quelconque des revendications 1 - 24, dans lequel on effecture une réaction de cyclisation impliquant un groupe méthylène, facultativement substitué, sur l'atome d'azote faisant partie de la structure hétérocyclique de la formule 1 à la revendication 1, l'atome de carbone de ce groupe méthylène étant activé en vue d'une telle cyclisation par, de préférence, un groupe epoxy, un atome d'halogène, ou une liaison oléfinique.

31. Utilisation des composés suivant une quelconque des revendications 1-24 comme agents de contraste pour des applications diagnostiques.

32. Utilisation des composés suivant une quelconque des revendications 1-24 pour fabriquer une formulation d'agent de contraste applicable en diagnostique

## Revendications (Revendications pour l'(les) Etat(s) contractant(s) suivant(s): ES)

1. Procédé pour préparer un composé de formule 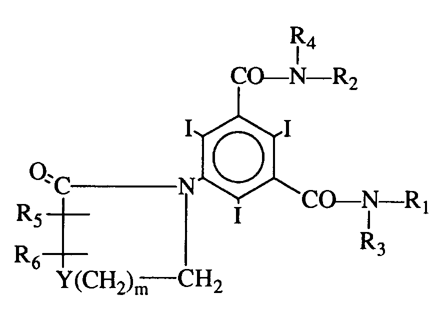 **dans laquelle** Y est une liaison simple, -CH₂-CH₂-, -CH₂O-, -OCH₂-, -N-CH₂-, -CH₂N-, -CH₂-H-CO-, -CH₂-, -O-, ou -N-;
R1 et R2, identiques ou différents, ont les formules: 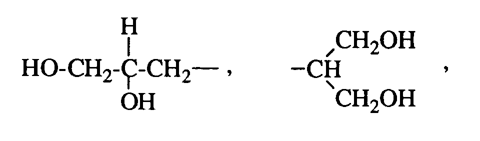 -CH₂CH₂OH 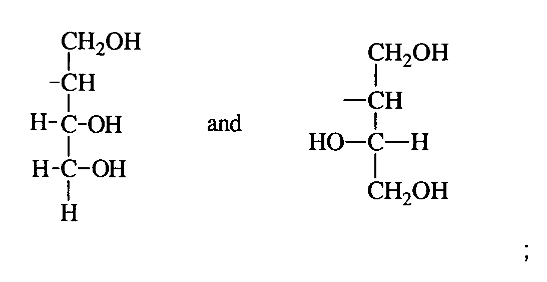 R3 et R4, identiques ou différents sont des restes hydrogène, méthyle, ou -CH₂CH₂OH; R5 et R6, identiques ou différents, sont des restes hydrogène, alcoyle, -CH₂CH₂OH, CH₂OH, ou OH, et m est 0 ou 1, étant donné qu'aucun atome de carbone méthénique ou méthinique de l'hétérocycle n'est simultanément relié à des atomes d'oxygène et d'azote, et étant encore donné que lorsque Y est une simple liaison, m n'est pas zéro, suivant lequel on effecture une réaction de cyclisation impliquant un groupe méthylène, facultativement substitué, sur l'atome d'azote faisant partie de la structure hétérocyclique de la formule 1, l'atome de carbone de ce groupe méthylène étant activé en vue d'une telle cyclisation par, de préférence, un groupe epoxy, un atome d'halogène, ou une liaison oléfinique.

2. Procédé suivant la revendication 1, où Y est une liaison simple.

3. Procédé suivant la revendication 1, où Y est -CH2CH2-.

4. Procédé suivant la revendication 1, où Y est -CH2O-.

5. Procédé suivant la revendication 1, où Y est -OCH2-.

6. Procédé suivant la revendication 1, où Y est -NCH2-.

7. Procédé suivant la revendication 1, où Y est -CH2N-.

8. Procédé suivant la revendication 1, où Y est -CH2-NCO-.

9. Procédé suivant la revendication 1, où Y est -CH2-.

10. Procédé suivant la revendication 1, où Y est -O-.

11. Procédé suivant la revendication 1, où Y est -N-.

12. Procédé suivant la revendication 1 où le produit est la N,N'-bis(2,3-dihydroxypropyl)-5[4-(hydroxy-méthyl)-2-oxo-3-oxazolodinyl]-2,4,6-triiodo-1,3-benzène-dicarboxamide suivant la revendication 1.

13. Procédé suivant la revendication 1 où le produit est la N,N'-bis(2,3-dihydroxypropyl)-5[4-(R)-(hydroxyméthyl)-2-oxo-3-oxazolinidyl]-2,4,6-triiodo-1,3-ben zènedicarboxamide suivant la revendication 1.

14. Procédé suivant la revendication 1 où le produit est la N,N'-bis[2-hydroxy-1-(hydroxyméthyl)éthyl]-2,4,6-triiodo-5-[4-(hydroxyméthyl)-2-oxo-3-oxazolinidyl]-1,3-benzènedicarboxamide suivant la revendication 1.

15. Procédé suivant la revendication 1 où le produit est la N,N'-bis[2-hydroxy-1-(hydroxyméthyl)éthyl]-5-[3-hydroxy-2-oxo-1-pyrrolidinyl]-2,4,6-triiodo-1,3-benzènedicarboxamide suivant la revendication 1.

16. Procédé suivant la revendication 1 où le produit est la N,N'-bis(2,3-dihydroxypropyl)-5-[3-hydroxy--2-oxo-1-pyrrolidinyl]-2,4,6-triiodo-1,3-benzène-dicarboxamide suivant la revendication 1.

17. Procédé suivant la revendication 1 où le produit est la N,N'-bis(2,3-dihydroxypropyl)-5[2-(hydroxy-méthyl)-5-oxo-1-pyrrolidinyl]-2,4,6-triiodo-1,3-benzène-dicarboxamide suivant la revendication 1.

18. Procédé suivant la revendication 1 où le produit est la N,N'-bis[2-hydroxy-1-(hydroxyméthyl)éthyl]-5-[3-hydroxyméthyl-5-oxo-4-morpholinyl]-2,4,6-triiodo-1,3-benzènedicarboxamide suivant la revendication 1.

19. Procédé suivant la revendication 1 où le produit est la N,N'-bis[2,3-dihydroxypropyl]-5-(3-hydroxy-2-oxo-1-piperidinyl)-2,4,6-triiodo-1,3-benzènedicarboxamide suivant la revendication 1.

20. Procédé suivant la revendication 1 où le produit est la N,N'-bis[2-hydroxy-1(hydroxyméthyl)éthyl-propyl]-5-(3-hydroxy-2-oxo-1-piperidinyl)-2,4,6-triiodo-1,3-benzènedicarboxamide suivant la revendication 1.

21. CompProcédé suivant la revendication 1 où le produit est la osé N,N'-bis[2,3-dihydroxy-1-propyl]-5-(3-hydroxy-2-oxo-1-pyrrolidinyl)-2,4,6-triiodo-1,3-benzènedicarboxamide suivant la revendication 1.

22. Procédé suivant la revendication 1 où le produit est la N,N'-bis[2-hydroxy-1(hydroxyméthyl)éthyl]-5-(2-hydroxyméthyl-5-oxo-1-pyrrolidinyl)-2,4,6-triiodo-1,3-benzènedicarboxamide suivant la revendication 1.

23. Procédé suivant la revendication 1 où le produit est la N,N'-bis[2,3-dihydroxypropyl]-5-[3-(hydroxyméthyl)-5-oxo-4-morpholinyl]-2,4,6-triiodo-1,3-benzènedicarboxamide suivant la revendication 1.

24. Procédé suivant la revendication 1 où le produit est la N,N'-bis[2,3-dihydroxypropyl]-5-(2-oxo-1-piperidinyl)-2,4,6-triiodo-1,3-benzèndicarboxamide suivant la revendication 1.

25. Utilisation des composés suivant une quelconque des revendications précédentes comme agent de contraste pour déterminer les conditions régnant à l'intérieur d'une cavité du corps.

26. Utilisation suivant la revendication 25, selon laquelle l'agent de contraste se trouve dans un milieu physiologiquement acceptable à une concentration de l'ordre d'environ 50 à 500 mg d'iode/ml.

27. Procédé de préparation de lactames par la réaction de dérivés 1-oxo-ω-alcoylénylamino-, ou 1-oxo-ω-oxaalcoylénamino- de 2,4,6-triodo-1,3-benzènedicarboxamides avec des agents iodants en présence d'une base.

28. Procédé suivant la revendication 27, dans lequel l'agent iodant est la N-iodosuccinimide.

29. Procédé suivant les revendications 27 ou 28, dans lequelles la base est l'hydroxyde de sodium, le méthylate de sodium ou l'hydrure de sodium.
